# EUROPEAN PATENT APPLICATION

(11) **EP 4 382 130 A1**
(43) Date of publication of application: **12.06.2024**
(21) Application number: 22857796.1
(22) Date of filing: 16.08.2022
(51) Int. Cl.: A61K 47/64, C07K 7/08, A61P 35/00

(54) **POLYPEPTIDE DRUG CONJUGATE HAVING NOVEL STRUCTURE AND APPLICATION THEREOF**

(30) Priority: 17.08.2021 CN 202110945285; 24.08.2021 CN 202110976710; 19.10.2021 CN 202111214465; 20.10.2021 CN 202111223632; 03.12.2021 CN 202111465873; 04.01.2022 CN 202210003460
(71) Applicant: Medshine Discovery Inc., Nanjing, Jiangsu 210032 (CN)
(72) Inventor: LI, Huining, Shanghai 200131 (CN); XIA, Jianhua, Shanghai 200131 (CN); JIANG, Zhigan, Shanghai 200131 (CN); HE, Haiying, Shanghai 200131 (CN); CHEN, Shuhui, Shanghai 200131 (CN)
(74) Representative: Bayramoglu et al.
(86) International application number: PCT/CN2022/112763
(87) International publication number: WO 2023/020485

(57) **Abstract**

Disclosed in the present invention are a novel polypeptide drug conjugate and an application thereof, and specifically disclosed is a compound represented by formula (V).

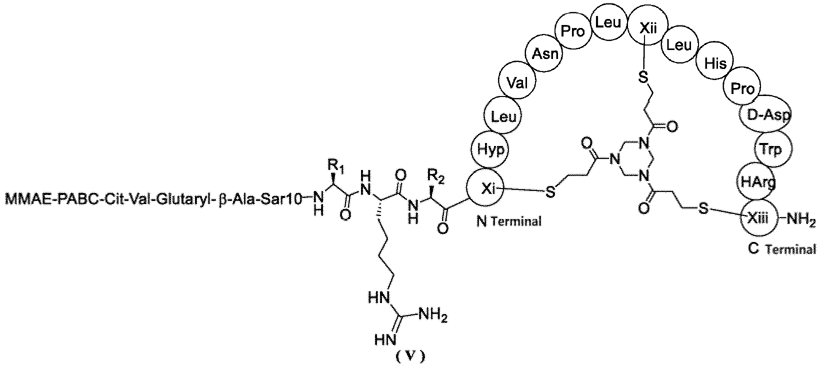

## Description

**The present invention claims the priority of the following applications:**
CN2021109452856, filed on August 17, 2021;
CN2021109767108, filed on August 24, 2021;
CN2021112144653, filed on October 19, 2021;
CN2021112236320, filed on October 20, 2021;
CN2021114658736, filed on December 3, 2021; and
CN2022100034604, filed on January 4, 2022.

### Technical Field

The present invention relates to a polypeptide drug conjugate with a novel structure and an application thereof, and specifically, to a compound represented by formula (V), or a pharmaceutically acceptable salt thereof.

### Background Art

In 2020, there were about 19,300,000 new cases of cancer and nearly 10,000,000 deaths worldwide. Although there are currently standardized treatments for most early-stage cancer patients, some cancer patients, especially those with advanced-stage cancer, have limited and unsatisfactory treatments due to disease progression and resistance or insensitivity to traditional treatments. Therefore, the research of tumor therapeutic drugs with new targets, mechanisms, and structures has always been an urgent problem to solve in the field of tumor therapy.

EphA2 is a newly emerging tumor-associated target with much attention in recent years, which has been reported to regulate the process of carcinogenesis and tumor progression. Unlike most Eph kinases, the distribution of EphA2 in adults is primarily restricted to rapidly proliferating epithelial cells. Studies have shown that EphA2 is overexpressed in a variety of cancers, such as prostate cancer, lung cancer, esophageal cancer, colorectal cancer, cervical cancer, ovarian cancer, and skin cancer. The high expression of EphA2 in tumors is directly associated with poor prognosis, high risk of metastasis, and shortened survival of cancer patients. The signaling pathway composed of EphA2 and its ligand Ephrin A1 induces the inhibition of various downstream kinases such as ERK and AKT, thereby regulating the migration, vitality, and proliferation of malignant cells. Therefore, EphA2 can serve as both a drug delivery target for tumor tissues and a target for tumor therapy.

### Summary of the Invention

The present invention provides a compound represented by formula (V), or a pharmaceutically acceptable salt thereof, 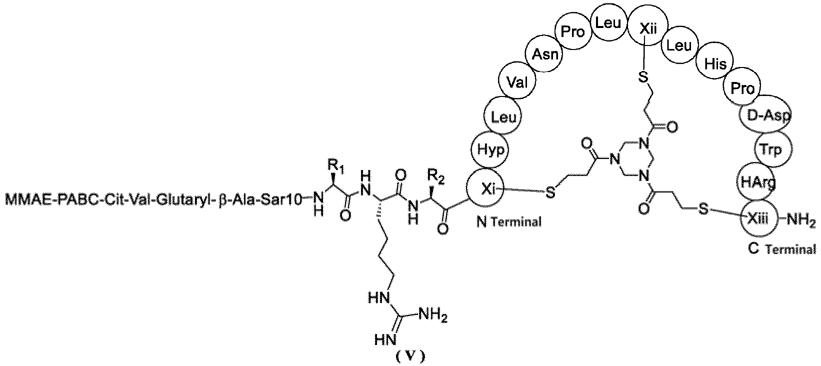 wherein 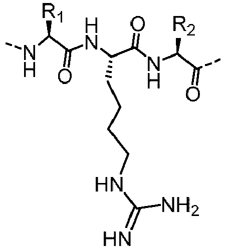 is selected from 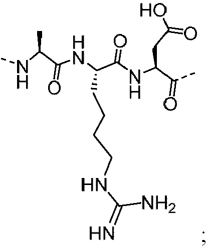 and
Xi, Xii, and Xiii are each independently selected from Cys, hCys, βCys, Pen, Dap, and N-methyl-Dap, Xi, Xii, and Xiii being not all Cys;
   or 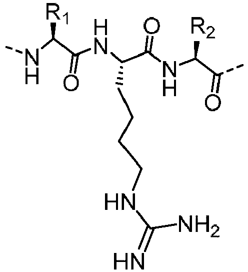 is selected from 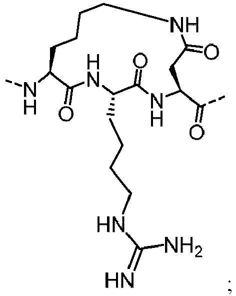
and Xi, Xii, and Xiii are each independently selected from Cys, hCys, PCys, Pen, Dap, and N-methyl-Dap.

The present invention provides a compound represented by formula (I), or a pharmaceutically acceptable salt thereof, 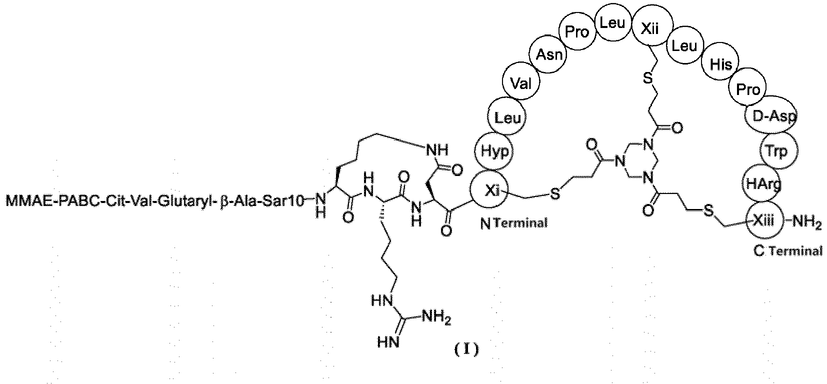 wherein
Xi, Xii, and Xiii are each independently selected from Cys, hCys, βCys, Pen, Dap, and N-methyl-Dap.

The present invention further provides a compound represented by formula (I'), or a pharmaceutically acceptable salt thereof, 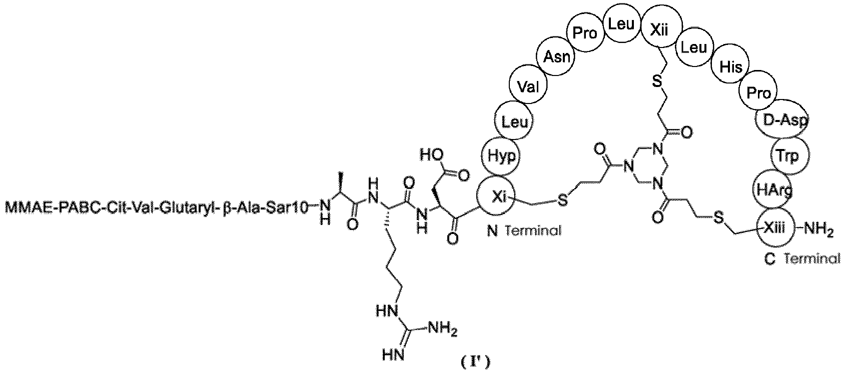 wherein
Xi, Xii, and Xiii are each independently selected from Cys, hCys, PCys, Pen, Dap, and N-methyl-Dap,
provided that Xi, Xii, and Xiii are not all Cys.

Some other variables of the present invention are derived from any combination of the above variables.

The present invention further provides compounds represented by the following formulas, or pharmaceutically acceptable salts thereof,

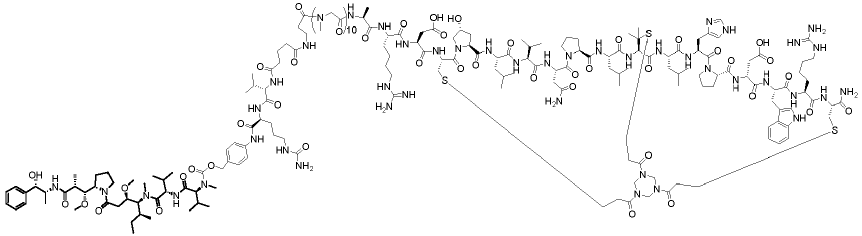

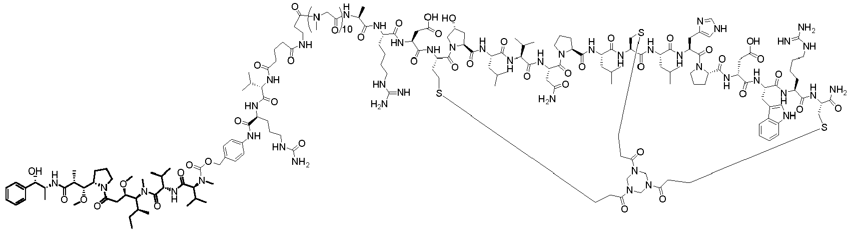

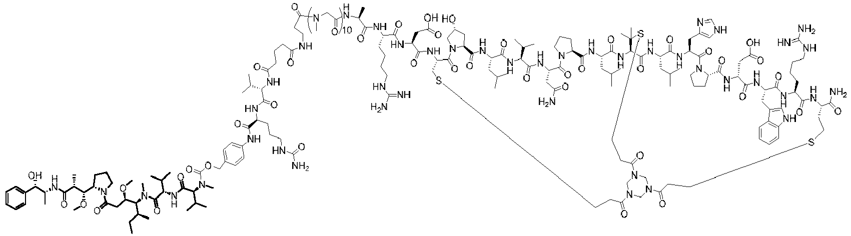

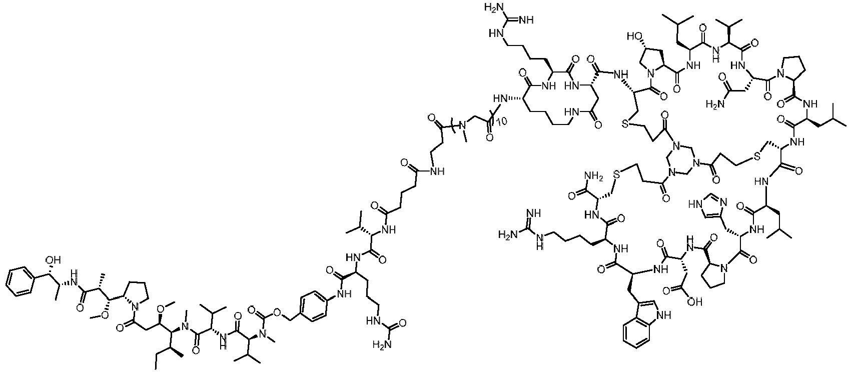

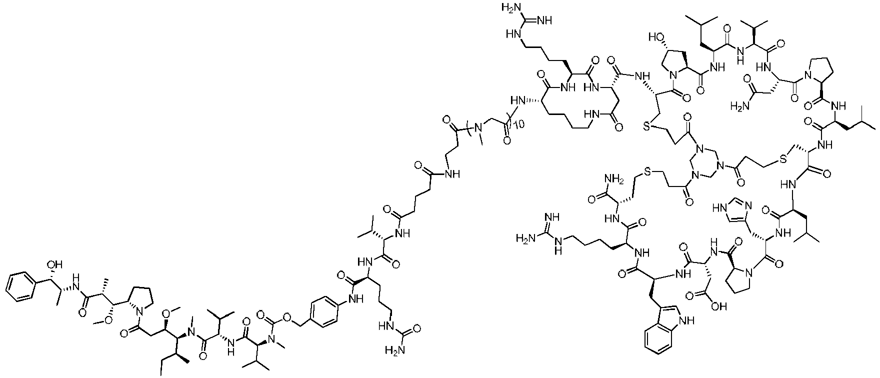

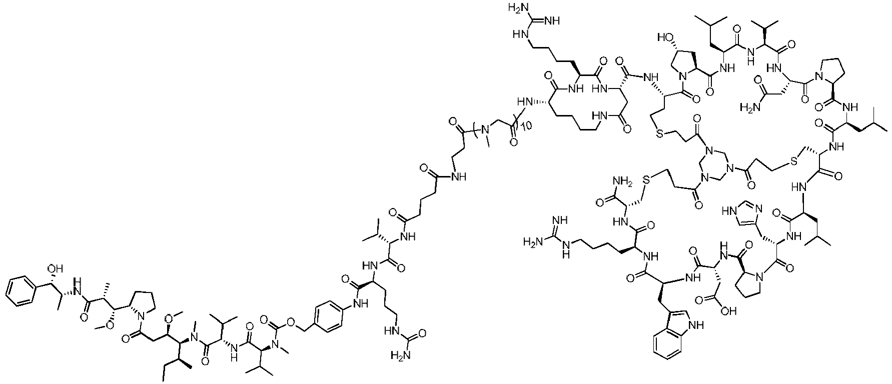

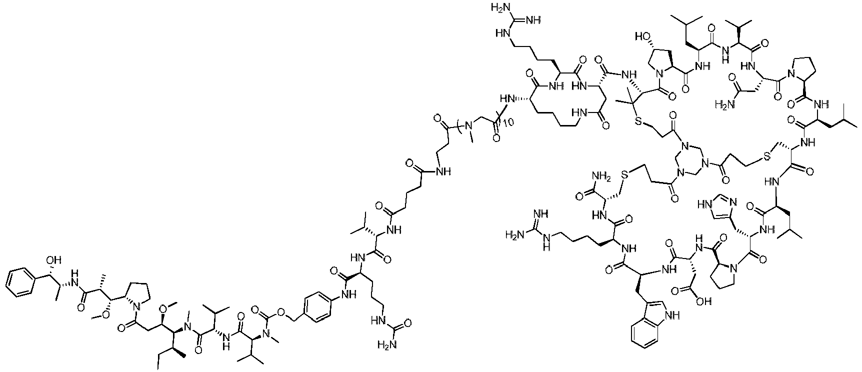

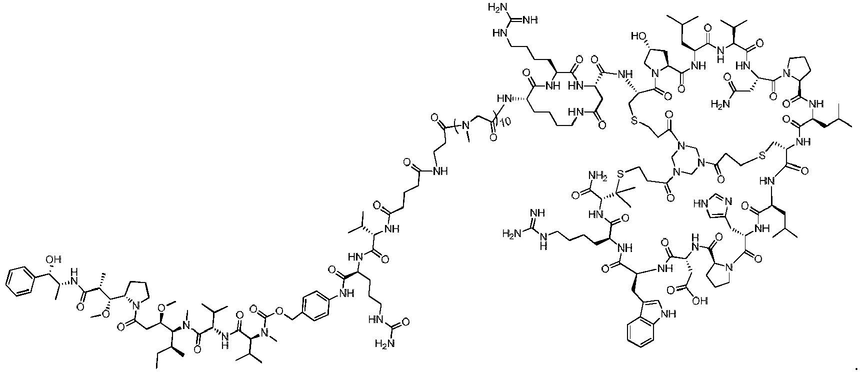

The present invention further provides the use of the compounds or the pharmaceutically acceptable salts thereof as described above in preparation of a drug for treating EphA2 overexpressed solid tumors.

The present invention further provides a method for treating EphA2 overexpressed solid tumors in a subject in need, comprising providing the subject with a therapeutically effective amount of the compounds or the pharmaceutically acceptable salts thereof as described above.

The present invention refers to the following preparation methods:

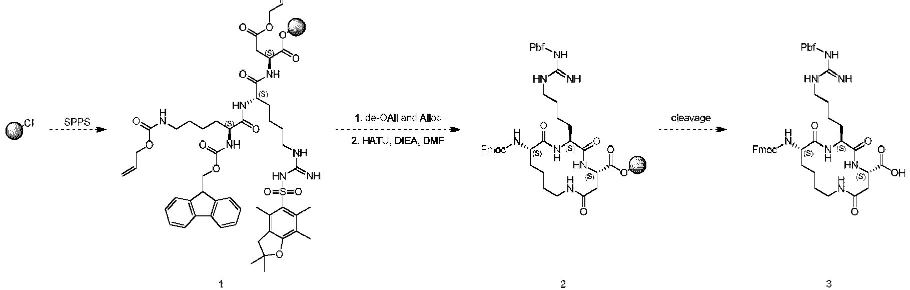

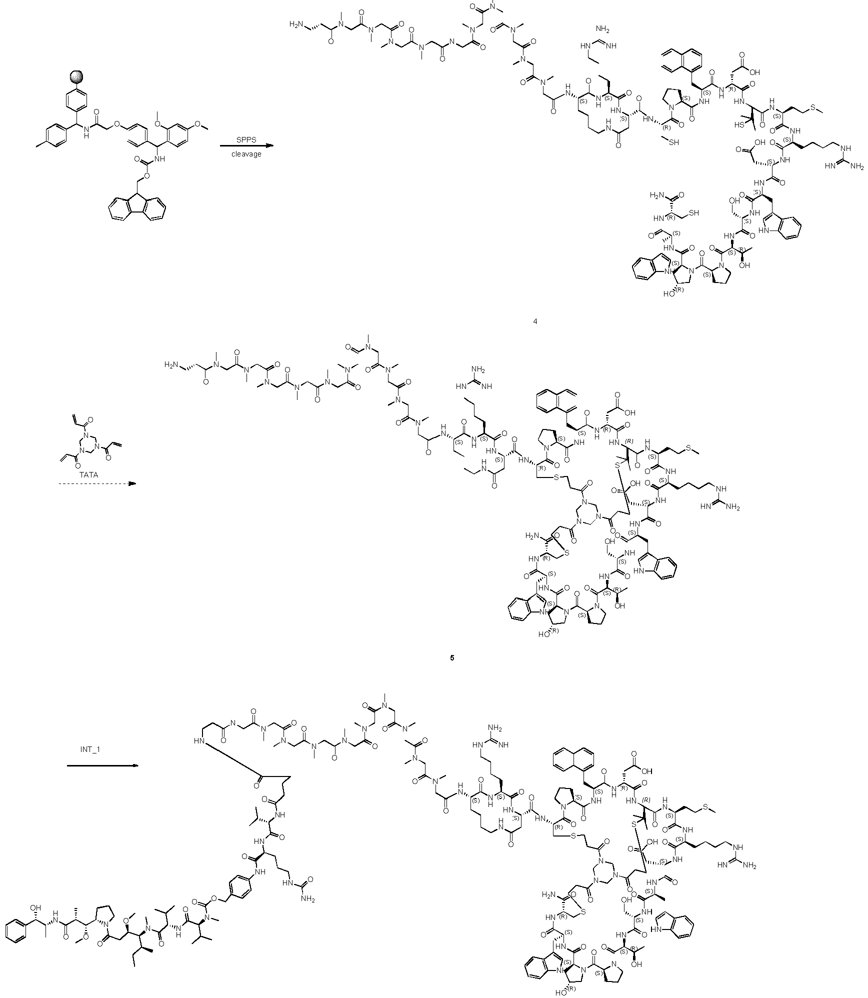

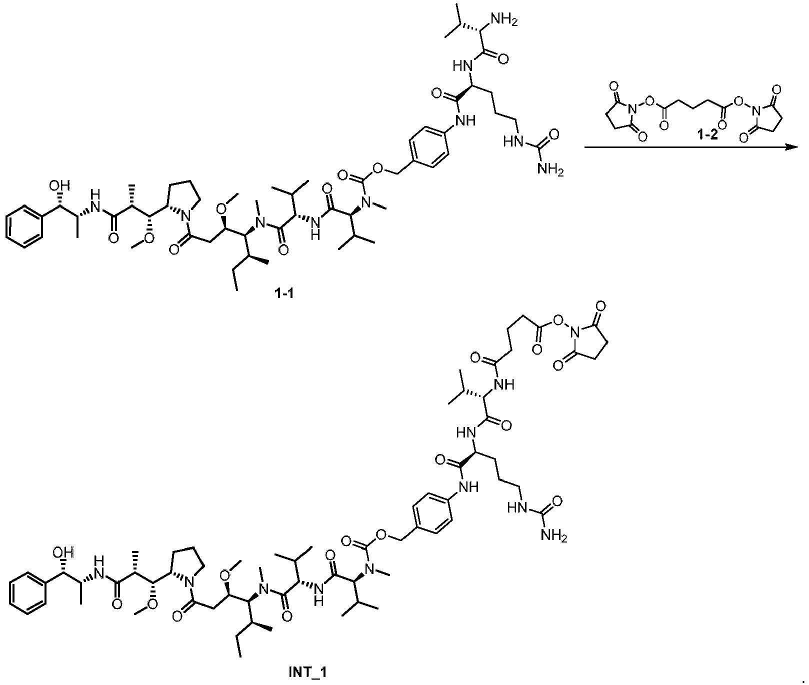

The present invention further provides the following test method.

### Test Method 1. Binding capacity test of the compounds of the present invention to EphA2 protein

### 1. Experimental purpose

To detect the affinity of the compound to be tested to the target protein EphA2 using the SPR method.

### 2. Materials and instruments

- Biacore 8K (GE Healthcare)
- 96-well Plate (Cat# 650101, greiner bio-one)
- CM5 chip (Cat# BR-1005-30, GE Healthcare)
- Amine Coupling Kit (Cat# BR-1000-50, GE Healthcare)
   EDC
   NHS
   1 M ethanolamine
- 10 mM sodium acetate pH 4.5 (Cat# BR-1003-50, GE Healthcare)
- DMSO (Cat# D4540, Sigma)
- P20 (Cat# BR-1000-54, GE Healthcare)
- PBS (Cat# BR-1006-72, GE Healthcare)
- EphA2 (Cat# 13926-H08HD, Sino Biological)

### 3. Experimental protocol

In this experiment, the amino coupling method is used. The target protein EphA2 is directly immobilized on the CM5 chip using Biacore 8K. The compound to be tested as an analyte is then diluted to a required concentration gradient with a buffer (10 mM PBS, pH 7.4, 137 mM NaCl, 2.7 mM KCl, 5% DMSO, and 0.05% P20) for multi-cycle kinetic detection, in which each cycle is formed by 180 seconds of sample injection and 180 seconds of dissociation, and then the next cycle is performed, to obtain kinetic analysis data on the affinity of the target protein EphA2 to the compound to be tested. The final data are subjected to Kinetics fitting analysis based on a 1:1 model using Biacore Insight Evaluation Software (V 2.0.15.12933).

### 4. Experimental method and procedure

1) Preparation of the buffer: 10 mM PBS, pH 7.4, 137 mM NaCl, 2.7 mM KCl, 5% DMSO, and 0.05% P20.
2) Activation of the CM5 chip: The CM5 chip is activated with 400 mM EDC and 100 mM NHS at a flow rate of 10 µL/min for 420 seconds.
3) Coupling of the target protein: The target protein is diluted to 10 µg/mL with 10 mM sodium acetate (pH 4.5) and coupled at a flow rate of 10 µL/min for 284 s. The 1#, 2#, and 3# channels on the chip are used in the experiment, and the coupling results are 1639.9RU, 1747.8RU, and 1702.2RU, respectively.
4) Blocking of the CM5 chip: The CM5 chip is blocked with 1 M ethanolamine at a flow rate of 10 µL/min for 420 seconds.
5) Analyte concentration: The compound to be tested is diluted with the buffer. The compound to be tested is diluted from 100 nM to 0.78 nM in a 2-fold gradient.
6) Sample injection analysis: Each concentration of the working solution of the compound to be tested corresponds to one cycle, with a flow rate of 30 µL/min for binding for 180 seconds and dissociating for 180 seconds. The last cycle is a 5% DMSO solvent correction cycle.
7) All results are subjected to kinetics fitting analysis based on a 1:1 model.

### TECHNICAL EFFECT

The compounds of the present invention have a strong binding effect to EphA2 and a significant inhibitory effect on the proliferation of cells cultured in vitro in tumor cell lines NCI-H1975 and SK-OV-3. The compounds of the present invention show a significant tumor growth inhibition effect in a human ovarian cancer SK-OV-3 cell subcutaneous xenograft tumor model and a human prostate cancer PC-3 subcutaneous xenograft tumor model, and both show a significant dose-dependence. The compounds of the present invention exhibit good pharmacokinetic properties, rapid clearance in plasma, low accumulation, low MMAE release, excellent in vitro stability in liver microsomes, plasma, and whole blood, and good druggability.

### Brief Description of the Drawings

FIG. 1 shows tumor growth curves of compounds of the present invention in a human prostate cancer cell subcutaneous xenograft tumor model.
FIG. 2 shows animal weight change curves of compounds of the present invention in a human prostate cancer cell subcutaneous xenograft tumor model.
FIG. 3 shows tumor growth curves of compounds of the present invention in a human ovarian cancer SK-OV-3 cell subcutaneous xenograft tumor BALB/c nude mouse model.
FIG. 4 shows animal weight change rates of compounds of the present invention in a human ovarian cancer SK-OV-3 cell subcutaneous xenograft tumor BALB/c nude mouse model.

### Definition and Description

Unless otherwise indicated, the following terms and phrases used herein are intended to have the following meanings. A particular term or phrase, unless specifically defined, should not be construed as indefinite or unclear but rather construed in a generic sense. When trade names appear herein, they are intended to refer to their corresponding goods or their active ingredients. The term "pharmaceutically acceptable" used herein refers to those compounds, materials, compositions, and/or dosage forms that are, within the scope of sound medical judgment, suitable for use in contact with the tissues of humans and animals without undue toxicity, irritation, allergic response, or other problems or complications, commensurate with a reasonable benefit/risk ratio. The term "pharmaceutically acceptable salt" refers to a salt of a compound of the present invention, prepared from the compound with the specified substituents found in the present invention and a relatively nontoxic acid or a base. When the compounds of the present invention contain relatively acid functional groups, base addition salts may be obtained by contacting such compounds with a sufficient amount of base in a neat solution or a suitable inert solvent. Pharmaceutically acceptable base addition salts include sodium, potassium, calcium, ammonium, organic amine, magnesium salts, or similar salts. When the compounds of the present invention contain relatively basic functional groups, acid addition salts may be obtained by contacting such compounds with a sufficient amount of acid in a neat solution or a suitable inert solvent. Certain specific compounds of the present invention contain basic and acid functional groups and thus can be converted to any base or acid addition salt.

The pharmaceutically acceptable salts of the present invention may be synthesized from a parent compound containing an acid radical or a basic group by conventional chemical methods. Generally, such salts are prepared by reacting these compounds in free acid or base form with a stoichiometric amount of appropriate base or acid in water, an organic solvent, or a mixture of both.

The "amino acid" refers to naturally occurring and synthetic amino acids, as well as amino acid analogs and amino acid mimetics that function similarly to naturally occurring amino acids. Naturally occurring amino acids are those encoded by the genetic code and those that have been later modified, such as hydroxyproline, gamma-carboxyglutamic acid, and O-phosphoserine. Amino acid analogs refer to compounds having the same basic chemical structure as naturally occurring amino acids (e.g., an alpha carbon bonded to a hydrogen, a carboxyl group, an amino group, and an R group), such as homoserine, norleucine, methionine sulfoxide, and methionine methylsulfonium. Such analogs may have modified R groups (e.g., norleucine) or modified peptide backbones but retain the same basic chemical structure as naturally occurring amino acids. The amino acid mimetics refer to chemical compounds that differ in structure from an amino acid's general chemical structure but function similarly to naturally occurring amino acids.

The therapeutic dose of the compounds of the present invention may depend, for example, on the following: the specific use of the treatment, the administration manner of the compounds, the health and condition of the patient, and the judgment of the prescribing physician. The proportion or concentration of the compounds of the present invention in a pharmaceutical composition may vary depending on a variety of factors, including dose, chemical characteristics (e.g., hydrophobicity), and the route of administration.

The term "treatment" refers to the administration of the compounds of the present invention or formulations to ameliorate or eliminate diseases or one or more symptoms associated with the diseases, including:
(i) inhibiting the diseases or disease states, i.e., controlling the development of the diseases or disease states;
(ii) alleviating the diseases or disease states, i.e., causing regression of the diseases or disease states.

The term "therapeutically effective amount" refers to an amount of the compounds of the present invention used to (i) treat a particular disease, condition, or disorder, (ii) relieve, ameliorate, or eliminate one or more symptoms of a particular disease, condition, or disorder, or (iii) prevent or delay the onset of one or more symptoms of the particular disease, condition, or disorder described herein. The amount constituting a "therapeutically effective amount" of the compounds of the present invention will vary depending on the compounds, the disease states and severity, the method of administration, and the age of the mammal to be treated, but it may routinely be determined by those skilled in the art based on their knowledge and the disclosure.

Unless otherwise requested, throughout the description and the appended claims, the word "comprise" and variations thereof, such as "comprises" and "comprising," should be interpreted as having an open and inclusive meaning, that is, as "including but not limited to."

Reference throughout the description to "one embodiment," "an embodiment," "in another embodiment," or "in certain embodiments" means the inclusion of specific referenced elements, structures, or characteristics related to the embodiment in at least one embodiment. Thus, the phrases "in one embodiment," "in an embodiment," "in another embodiment," or "in certain embodiments" that appear in various places throughout the description do not necessarily all refer to the same embodiment. In addition, the specific elements, structures, or characteristics may be combined in one or more embodiments in any appropriate manner.

Unless otherwise indicated, the term "isomers" is meant to include geometric isomers, cis-trans isomers, stereoisomers, enantiomers, optical isomers, diastereomers, and tautomers.

The compounds of the present invention may exist in specific geometric or stereoisomeric forms.

The present invention contemplates all such compounds, including cis and trans isomers, (-)- and (+)-enantiomers, (*R*)- and (*S*)-enantiomers, diastereomers, (*D*)-isomers, (*L*)-isomers, and racemic and other mixtures thereof, such as mixtures enriched with enantiomers or diastereomers, all of which are within the scope of the present invention. Additional asymmetric carbon atoms may be present in substituents such as an alkyl group. All such isomers and mixtures thereof are included within the scope of the present invention.

Unless otherwise indicated, the term "enantiomers" or "optical isomers" refers to stereoisomers that are mirror images of one another.

Unless otherwise indicated, the term "cis-trans isomers" or "geometric isomers" results from the inability of a double bond or a single bond of a ring-forming carbon atom to rotate freely.

Unless otherwise indicated, the term "diastereomer" refers to stereoisomers in which the molecules have two or more chiral centers and are not mirror images of each other.

Unless otherwise indicated, "(+)" means dextrorotatory, "(-)" means levorotatory, and "(±)" means racemic.

Unless otherwise indicated, the absolute configuration of a stereocenter is represented by wedge-shaped solid bonds ( 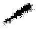 ) and wedge-shaped dashed bonds ( 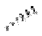 ), and the relative configuration of the stereocenter is represented by straight solid bonds ( 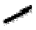 ) and straight dashed bonds ( 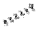 ); the wedge-shaped solid bonds ( 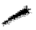 ) or the wedge-shaped dashed bonds ( 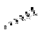 ) is represented by wavy lines ( 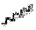 ), or the straight solid bonds ( 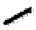 ) or the straight dashed bonds ( 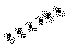 ) is represented by wavy lines ( 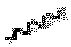 ).

Unless otherwise indicated, the terms "enriched in one isomer," "isomerically enriched," "enriched in one enantiomer," or "enantiomerically enriched" mean that one isomer or enantiomer is present in less than 100% and greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise indicated, the term "isomer excess" or "enantiomeric excess" refers to the difference between the relative percentages of two isomers or two enantiomers. For example, where the content of one isomer or enantiomer is 90% and the content of the other isomer or enantiomer is 10%, the isomer or enantiomer excess (ee value) is 80%.

Optically active (R)- and (S)-isomers, as well as D and L isomers, may be prepared by chiral synthesis, chiral reagents, or other conventional techniques. A desired enantiomer of a compound of the present invention may be prepared by asymmetric synthesis or derivatization with chiral auxiliary agents in which the resulting diastereomeric mixture is separated and the auxiliary group is cleaved to provide the pure desired enantiomer. Alternatively, when the molecule contains a basic functional group, such as an amino group, or an acid functional group, such as a carboxyl group, a diastereomeric salt is formed with an appropriate optically active acid or base, followed by diastereomeric resolution by conventional methods well known in the art, followed by recovery to obtain the pure enantiomer. In addition, the enantiomers and diastereomers are typically separated using chromatography employing a chiral stationary phase, optionally in combination with chemical derivatization (e.g., carbamate formation from an amine).

The compounds of the present invention may contain unnatural proportions of atomic isotopes on one or more atoms constituting the compounds. For example, the compounds may be labeled with radioisotopes, such as tritium (³H), iodine-125 (¹²⁵I), or C-14 (¹⁴C). Also, for example, a deuterated drug may be formed by replacing hydrogen with deuterium, wherein the bond formed between deuterium and carbon is stronger than that formed between ordinary hydrogen and carbon, and the deuterated drug has the advantages of reduced toxic side effects, increased drug stability, enhanced therapeutic efficacy, and prolonged biological half-life, compared with the undeuterated drug. All isotopic variations of the compounds of the present invention, whether radioactive or not, are included within the scope of the present invention.

When the attachment group listed does not indicate the attachment direction thereof, the attachment direction is arbitrary. For example 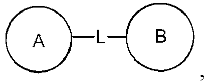 when the attachment group L is -M-W-, the -M-W- may be attached to ring A and ring B in the same direction as the reading order from left to right to form 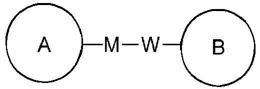 or may be attached to ring A and ring B in the opposite direction as the reading order from left to right to form 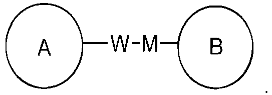 Combinations of the attachment group, the substituents, and/or variants thereof are permissible only if such combinations result in stable compounds.

Unless otherwise specified, when a group has one or more attachable sites, any one or more of the sites of the group may be attached to other groups by chemical bonds. When the chemical bonds are attached in a non-positional manner and there are H atoms at the attachable sites, then the number of H atoms at the sites will correspondingly decrease as a function of the number of attached chemical bonds to become a group of corresponding valence when the chemical bonds are attached. The chemical bonds attaching the sites to other groups may be represented by straight solid bonds ( 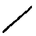 ), straight dashed bonds ( 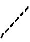 ), or wavy lines ( 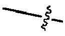 ). For example, the straight solid bond in -OCH₃ represents the attachment to another group through the oxygen atom of the group; the straight dashed bonds in 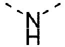 represent the attachment to other groups through both ends of the nitrogen atom of the group; the wavy lines in 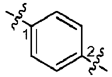 represent the attachment to other groups through the carbon atoms of the phenyl group at position 1 and 2.

Unless otherwise specified, the term "C₁₋₄ alkyl" represents a straight or branched saturated hydrocarbon group consisting of 1 to 4 carbon atoms. The C₁₋₄ alkyl includes C₁₋₂ alkyl, C₁₋₃ alkyl, C₂₋₃ alkyl, and the like, which may be monovalent (e.g., methyl), divalent (e.g., methylene), or polyvalent (e.g., methine). Examples of C₁₋₄ alkyl groups include, but are not limited to, methyl (Me), ethyl (Et), propyl (including n-propyl and isopropyl), butyl (including n-butyl, isobutyl, s-butyl, and t-butyl), and the like.

Unless otherwise indicated, amino acid X in the present invention is attached to TATA through the thiol group on the amino acid residue. For example, when Xi is Pen, 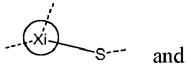 and 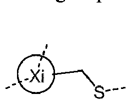 both represent 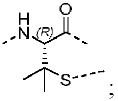 when Xi is hCys, 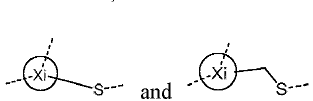 both represent 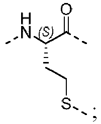 when Xi is βCys, 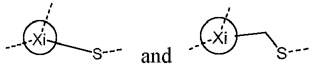 both represent

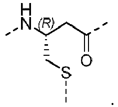

The compounds of the present invention may be structurally confirmed by conventional methods well known to those skilled in the art. If the present invention relates to the absolute configuration of the compounds, the absolute configuration may be confirmed by conventional technical means in the art. For example, for single crystal X-ray diffractometry (SXRD), the diffraction intensity data of the cultured single crystal is collected using Bruker D8 venture diffractometer, with a CuKa radiation as light source and a ϕ/ω scanning mode, and the absolute configuration may be confirmed by further resolving the crystal structure using a direct method (Shelxs97) after collecting relevant data.

The compounds of the present invention may be prepared by various synthetic methods well known to those skilled in the art, including the specific embodiments set forth below, embodiments formed in combination with other chemical synthetic methods, and equivalents well known to those skilled in the art. Preferred embodiments include, but are not limited to, the examples of the present invention.

The compounds are named according to conventional nomenclature in the art or using ChemDraw^{®} software, and the commercially available compounds adopt the name in the supplier catalog.

The solvent used in the present invention is commercially available.

The following abbreviations are used in the present invention: eq. for equivalent; SPPS for polypeptide solid phase synthesis; TFA for trifluoroacetic acid; DIEA for diisopropylethyl amine; DMF for N,N-dimethylformamide; HATU for 2-(7-azabenzotriazole)-N,N,N',N'-tetramethyluronium hexafluorophosphate; EDC for 1-(3-dimethylaminopropyl)-3-ethylcarbodiimide hydrochloride; NHS for N-hydroxysuccinimide; TIS for triisopropylsilane; DTT for DL-1,4-dithiothreitol; MMAE for monomethyl auristatin E, with a structure of 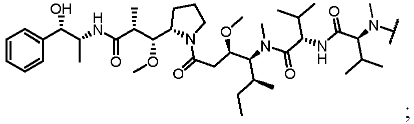 PABC for 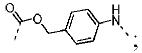 Cit for L-citrulline; Val for L-valine; Glutaryl for 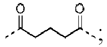 β-Ala for 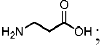 Sar for 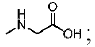 Sar10 for 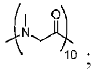 Cys for L-cysteine; hCys for 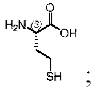 PCys for 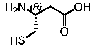 ; Pen for 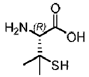 ; N-methyl-Dap for 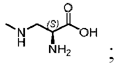 1Nal for 1-naphthylalanine; hArg or HArg for L-homoarginine; Hyp for L-hydroxyproline; Trp for L-tryptophan; Pro for L-proline; Thr for L-threonine; Ser for L-serine; Asp for L-aspartic acid; dAsp or D-Asp for D-aspartic acid; Fmoc for 9-fluorenylmethyloxycarbonyl; Boc for tert-butoxycarbonyl; Trt for trityl; Pbf for 2,2,4,6,7-pentamethyldihydrobenzofuran-5-sulfonyl; and PBS for phosphate buffer.

### Detailed Description of the Invention

The following examples describe the present invention in detail, but they are not meant to impose any unfavorable limitation on the present invention. The present invention has been described in detail herein, and its specific embodiments are also disclosed. It will be apparent to those skilled in the art that various changes and modifications can be made to the specific embodiments without departing from the spirit and scope of the present invention.

### Example 1

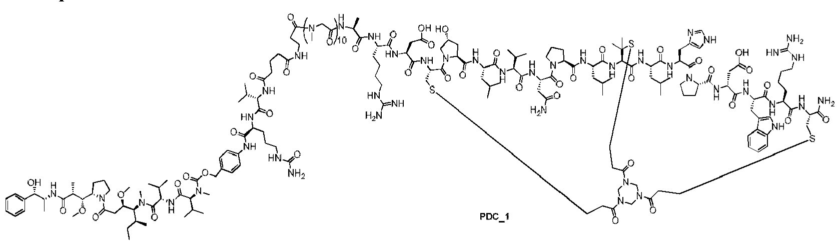

### Synthetic route:

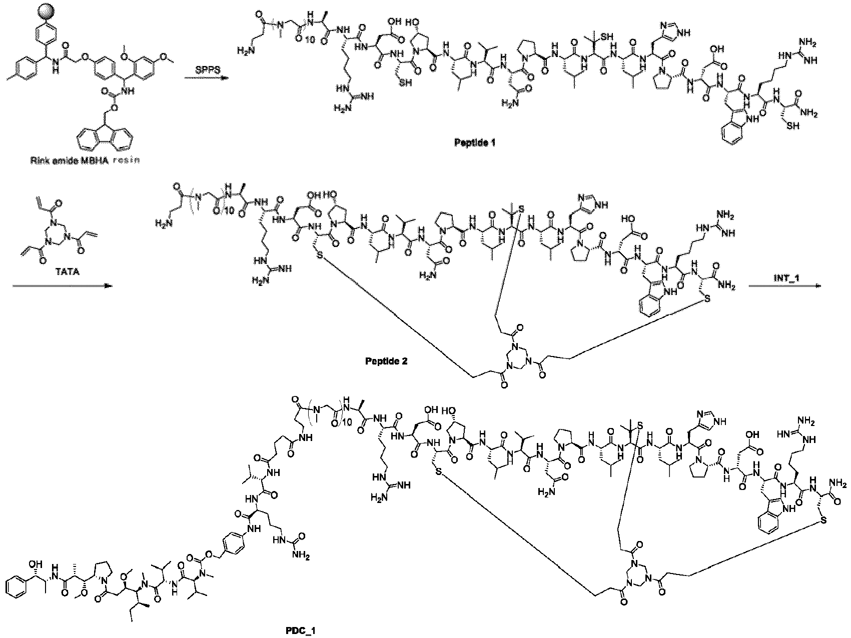

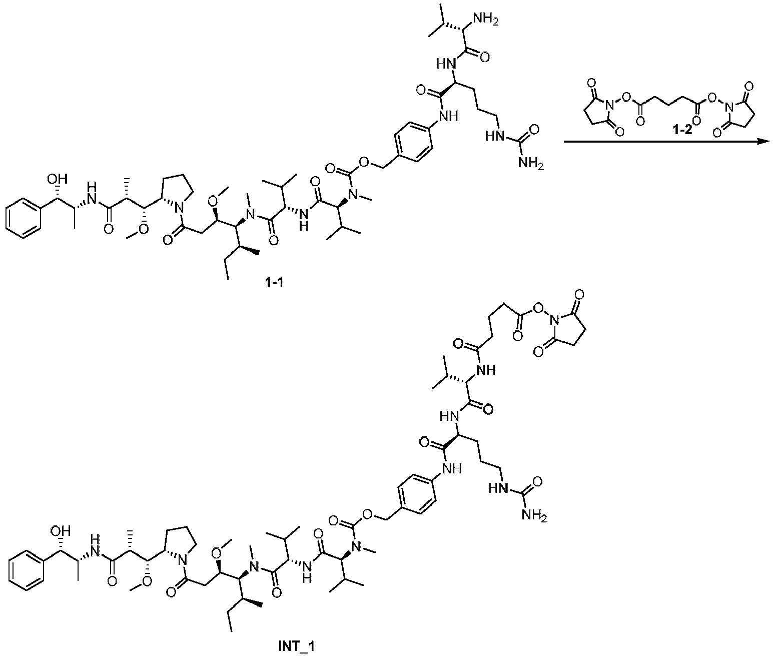

### Step 1: Synthesis of a TFA salt of Peptide 1

The polypeptide was synthesized using the standard step-wise synthesis method.
1) DMF (20 mL) was added to a container containing Rink amide MBHA resin (0.5 mmol, 1.85 g, with 0.27 mmol/g of a substrate), and the resin was allowed to swell for 2 hours.
2) The obtained mixture was drained and then rinsed three times with DMF, with nitrogen sparging for 30 seconds each time.
3) A 20% piperidine/DMF was added, and then the system reacted for 30 minutes.
4) The obtained mixture was drained and then rinsed five times with DMF, with nitrogen sparging for 30 seconds each time.
5) A Fmoc-protected amino acid solution was added, and the system was sparged with nitrogen for 30 seconds. A condensing agent was then added, and the system reacted under N₂ sparging for approximately 1 hour.
6) Steps 2 to 5 were repeated to condense the next amino acid.

**Table 1. Addition order**

| # | **Raw material** | **Condensing agent** |
|---|---|---|
| 1 | Fmoc-Cys(Trt)-OH (3.0 eq.) | HATU (2.85 eq.) and DIEA (6.0 eq.) |
| 2 | Fmoc-hArg(Pbf)-OH (2.0 eq.) | HATU (1.90 eq.) and DIEA (4.0 eq.) |
| 3 | Fmoc-Trp(Boc)-OH (3.0 eq.) | HATU (2.85 eq.) and DIEA (6.0 eq.) |
| 4 | Fmoc-dAsp(OtBu)-OH (3.0 eq.) | HATU (2.85 eq.) and DIEA (6.0 eq.) |
| 5 | Fmoc-Pro-OH (3.0 eq.) | HATU (2.85 eq.) and DIEA (6.0 eq.) |
| 6 | Fmoc-His(Trt)-OH (3.0 eq.) | HATU (2.85 eq.) and DIEA (6.0 eq.) |
| 7 | Fmoc-Leu-OH (3.0 eq.) | HATU (2.85 eq.) and DIEA (6.0 eq.) |
| 8 | Fmoc-Pen(Trt)-OH (3.0 eq.) | HATU (2.85 eq.) and DIEA (6.0 eq.) |
| 9 | Fmoc-Leu-OH (3.0 eq.) | HATU (2.85 eq.) and DIEA (6.0 eq.) |
| 10 | Fmoc-Pro-OH (3.0 eq.) | HATU (2.85 eq.) and DIEA (6.0 eq.) |
| 11 | Fmoc-Asn(Trt)-OH (3.0 eq.) | HATU (2.85 eq.) and DIEA (6.0 eq.) |
| 12 | Fmoc-Val-OH (3.0 eq.) | HATU (2.85 eq.) and DIEA (6.0 eq.) |
| 13 | Fmoc-Leu-OH (3.0 eq.) | HATU (2.85 eq.) and DIEA (6.0 eq.) |
| 14 | Fmoc-Hyp(tBu)-OH (3.0 eq.) | HATU (2.85 eq.) and DIEA (6.0 eq.) |
| 15 | Fmoc-Cys(Trt)-OH (3.0 eq.) | HATU (2.85 eq.) and DIEA (6.0 eq.) |
| 16 | Fmoc-Asp(OtBu)-OH (3.0 eq.) | HATU (2.85 eq.) and DIEA (6.0 eq.) |
| 17 | Fmoc-hArg(Pbf)-OH (2.0 eq.) | HATU (1.90 eq.) and DIEA (4.0 eq.) |
| 18 | Fmoc-Ala-OH (3.0 eq.) | HATU (2.85 eq.) and DIEA (6.0 eq.) |
| 19 | Fmoc-Sar-OH (5.0 eq.) | HATU (4.75 eq.) and DIEA (10.0 eq.) |
| 20 | Fmoc-Sar-OH (5.0 eq.) | HATU (4.75 eq.) and DIEA (10.0 eq.) |
| 21 | Fmoc-Sar-OH (5.0 eq.) | HATU (4.75 eq.) and DIEA (10.0 eq.) |
| 22 | Fmoc-Sar-OH (5.0 eq.) | HATU (4.75 eq.) and DIEA (10.0 eq.) |
| 23 | Fmoc-Sar-OH (5.0 eq.) | HATU (4.75 eq.) and DIEA (10.0 eq.) |
| 24 | Fmoc-Sar-OH (5.0 eq.) | HATU (4.75 eq.) and DIEA (10.0 eq.) |
| 25 | Fmoc-Sar-OH (5.0 eq.) | HATU (4.75 eq.) and DIEA (10.0 eq.) |
| 26 | Fmoc-Sar-OH (5.0 eq.) | HATU (4.75 eq.) and DIEA (10.0 eq.) |
| 27 | Fmoc-Sar-OH (5.0 eq.) | HATU (4.75 eq.) and DIEA (10.0 eq.) |
| 28 | Fmoc-Sar-OH (5.0 eq.) | HATU (4.75 eq.) and DIEA (10.0 eq.) |
| 29 | Fmoc-β-Ala-OH (3.0 eq.) | HATU (2.85 eq.) and DIEA (6.0 eq.) |

### Polypeptide cleavage and purification:

1) A cleavage buffer (90% TFA/2.5% TIS/2.5% H₂O/5.0% DTT) was added to a flask containing the side chain-protected polypeptide and stirred at room temperature for 2 hours.
2) The polypeptide was sedimented with ice isopropyl ether and centrifuged using a centrifuge (3000 rpm, 3 mins).
3) The obtained polypeptide was washed twice more with isopropyl ether.
4) The crude polypeptide was dried to give the TFA salt of compound **Peptide 1.**

### Step 2: Synthesis of an acetate salt of Peptide 2

The crude TFA salt of **Peptide 1** (1.40 g, 470 µmοl) was dissolved in 50% MeCN/H₂O (500 mL), and TATA (140 mg, 560 µmοl) was slowly added to the stirred solution at room temperature. The reaction mixture was stirred at room temperature for 30 minutes, and then the pH was adjusted to 8 with NH₄HCO₃. The reaction was stirred at room temperature for an additional 12 hours. When LCMS showed that the reaction was complete, stirring was stopped. The mixture was purified by reverse phase preparation (A: 0.075% TFA in H₂O, B: CH₃CN) to give the acetate salt of **Peptide 2**.

**Table 2. Purification conditions**

| Separation conditions | | |
|---|---|---|
| Dissolution condition | Dissolved in 20% MeCN/H₂O | |
| Machine model | SHIMADZU LC-8A | |
| Mobile phase | A: H₂O (0.075% TFA/H₂O) | |
| | B CH₃CN | |
| Separation gradient | 19-39%-40 min, retention time: 20 min | |
| Column type | Welch Ultimate^{®} XB-C18, 250*50 mm, 10 µm, 120Å + Welch | |
| | Xtimate^{®}C18, 250*50 mm, 10 µm, 120Å | |
| Flow rate | 80 mL/min | |
| Wavelength | 214/254 nm | |
| Temperature | 25°C | |

| Second purification and separation conditions | | |
|---|---|---|
| Dissolution condition | | Dissolved in 20% MeCN/H₂O |
| Machine model | | Gilson GX-281A |
| Mobile phase | | A: H₂O (0.5% AcOH/H₂O) |
| | B CH₃CN | |
| Separation gradient | 13-33%-40 min, retention time: 22 min | |
| Column type | YMC-Actus Triart C18, 250*30 mm, 5 µm, 120Å | |
| Flow rate | 20 mL/min | |
| Wavelength | 214/254 nm | |
| Temperature | 25°C | |

The synthesis of acetate salts of the other two polypeptides, **Peptide 3** and **Peptide 4,** was similar to that of the acetate salt of **Peptide 2**.

**Table 3. Polypeptide sequences of Peptide 2 to 4**

| Comp. No. | Polypeptide sequence |
|---|---|
| **Peptide 2** | H-β-Ala-Sar10-Ala-hArg-Asp-Cys-Hyp-Leu-Val-Asn-Pro-Leu-Pen-Leu-His-Pro- dAsp-Trp-hArg-Cys-NH2,TATA |
| **Peptide 3** | H-β-Ala-Sar10-Ala-hArg-Asp-hCys-Hyp-Leu-Val-Asn-Pro-Leu-Cys-Leu-His-Pr o-dAsp-Trp-hArg-Cys-NH2,TATA |
| **Peptide 4** | H-β-Ala-Sar10-Ala-hArg-Asp-Cys-Hyp-Leu-Val-Asn-Pro-Leu-Pen-Leu-His-Pro- dAsp-Trp-hArg-hCys-NH2,TATA |

### Step 3: Synthesis of a TFA salt of intermediate INT_1

Compound **1-1** (200.0 mg, 178.0 µmοl) was dissolved in DMF (5 mL), and DIEA (31.0 µL, 178.0 µmοl) was added at 0°C and stirred for 10 mins. Compound **1-2** (290.4 mg, 890.1 µmοl) was dissolved in DMF (5 mL) in another reaction flask and stirred at 0°C for 10 mins. The reaction solution of compound **1-1** was then dropwise added to the stirred reaction solution of compound **1-2** at 0°C and stirred at 0°C for 30 mins. The reaction solution was filtered to remove insoluble residues. The filtrate was directly purified by reverse phase preparation (TFA system) to give the TFA salt of compound **INT_1**.

### Step 4: Synthesis of an acetate salt of PDC_1

The TFA salt of polypeptide compound **peptide 2** (87.0 mg, 27.1 µmοl) was dissolved in DMF (0.5 mL), and then DIEA (12.9 uL, 74.4 µmοl) was added and stirred at room temperature for 10 mins. Next, the TFA salt of compound **INT_1** (36.2 mg, 27.1 µmοl) was dissolved in DMF (0.3 mL), dropwise added to the above reaction solution of the TFA salt of polypeptide compound **peptide 2**, and then stirred at room temperature for 2 hours. When LC-MS showed that the reaction was complete, the reaction was stopped. The reaction solution was filtered to remove insoluble residues. The filtrate was directly purified by reverse phase preparation (TFA system), lyophilized, and then converted to an AcOH salt by preparation to give the acetate salt of **PDC_1.**

### Example 2

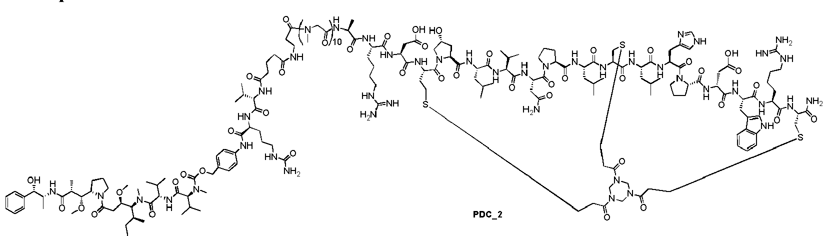

### Example 3

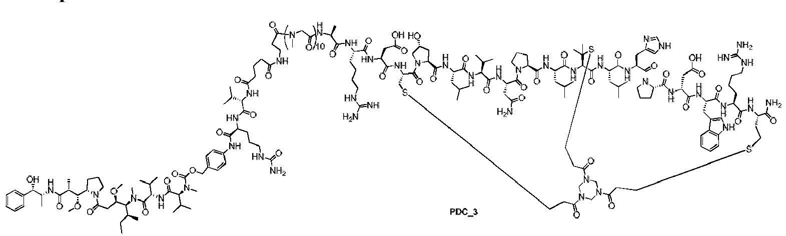

An acetate salt of PDC_2 and an acetate salt of PDC_3 were prepared in the same manner as the synthesis of the acetate salt of PDC _1.

**Table 4. Structures and mass spectrometry data of PDC_1 to 3**

| **Exampl e** | **Compou nd No.** | **Raw material** | **PDC compound structure** | **Mass (experimen tal value) measured value)** | **Molecul ar weight (calculat ed value)** |
|---|---|---|---|---|---|
| **1** | **PDC_1** | **Peptide 2** | MMAE-PABC-Cit-Val-Glutaryl-β-Ala-Sar10- Ala-hArg-Asp-Cys-Hyp-Leu-Val-Asn-Pro-Le u-Pen-Leu-His-Pro-dAsp-Trp-hArg-Cys-NH2, TATA | 1477.6 (M+3H⁺)/3 | 4429.8 |
| **2** | **PDC_2** | **Peptide 3** | MMAE-PABC-Cit-Val-Glutaryl-β-Ala-Sar10- Ala-hArg-Asp-hCys-Hyp-Leu-Val-Asn-Pro-L eu-Cys-Leu-His-Pro-dAsp-Trp-hArg-Cys-NH | 1104.9 (M+4H⁺)/4 | 4415.6 |
| | | | ₂,TATA | | |
| **3** | **PDC_3** | **Peptide 4** | MMAE-PABC-Cit-Val-Glutaryl-β-Ala-Sar10- Ala-hArg-Asp-Cys-Hyp-Leu-Val-Asn-Pro-Le u-Pen-Leu-His-Pro-dAsp-Trp-hArg-hCys-NH 2,TATA | 1112.1 (M+4H⁺)/4 | 4444.4 |

### Example 4

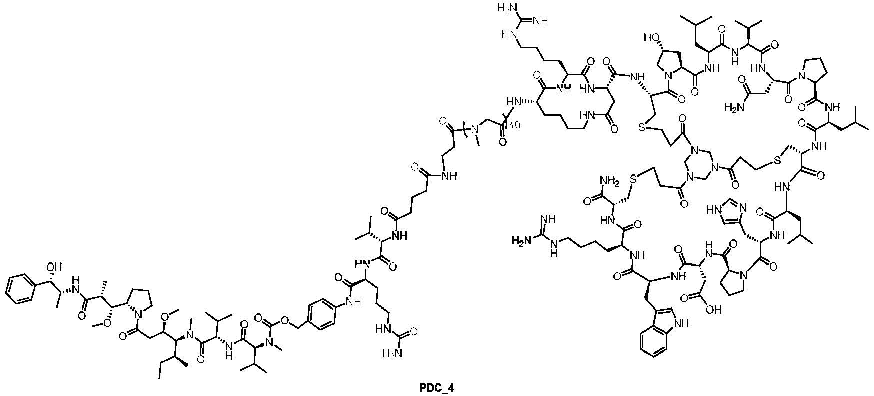

### Synthetic route:

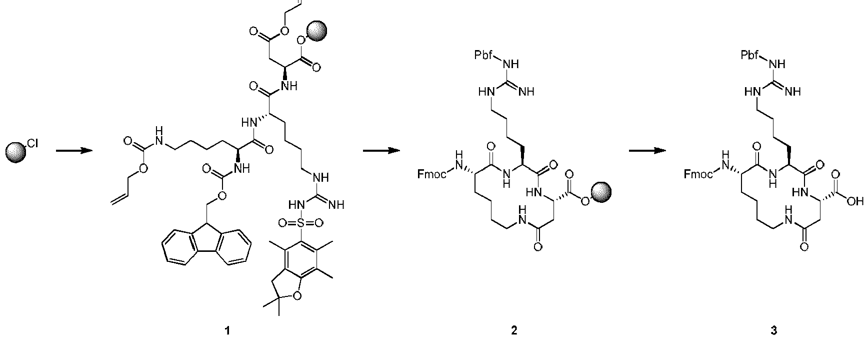

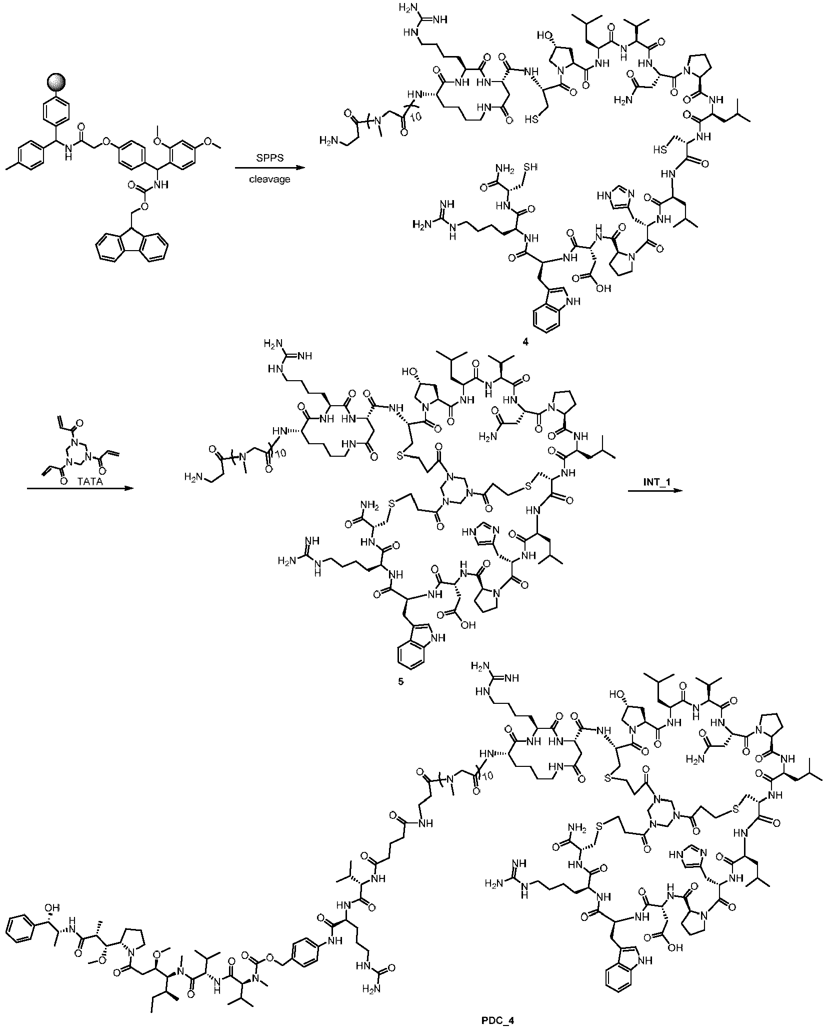

### 1. Synthesis of intermediate 3:

### Polypeptide synthesis:

The polypeptide was synthesized using the standard step-wise synthesis method.
1) DCM was added to a container containing CTC Resin (10.0 mmol, 10.0 g) and Fmoc-Asp(OAll)-OH.
2) DIEA (4.00 eq) was added and stirred for 2 hours.
3) MeOH (10.0 mL) was added and stirred for 30 minutes.
4) The obtained mixture was drained and then rinsed three times with DMF, with nitrogen sparging for 30 seconds each time.
5) A 20% piperidine/DMF was added, and then the system reacted for 30 minutes.
6) The obtained mixture was drained and then rinsed five times with DMF, with nitrogen sparging for 30 seconds each time.
7) A Fmoc-protected amino acid solution was added, and the system was sparged with nitrogen for 30 seconds. A condensing agent was then added, and the system reacted under N2 sparging for approximately 1 hour.
8) Steps 4 to 7 were repeated to condense the next amino acid until the Fmoc-Lys(Alloc)-OH attachment was completed.
9) The obtained mixture was drained and then rinsed three times with DMF and three times with DCM, with nitrogen sparging for 30 seconds each time.
10) Remove of OAll and Alloc: Pd(PPh₃)₄ (0.1 eq) and PhSiH₃ (10.0 eq) were added to the DCM resin solution, and the system reacted under N₂ sparging for approximately 15 minutes and drained. This step was repeated three times.
11) The obtained mixture was drained and then rinsed five times with DMF, with nitrogen sparging for 30 seconds each time.
12) Cyclization: The condensing agent solution HATU (2.85 eq.) and DIEA (6.0 eq.) were added, and the system reacted under N₂ sparging for approximately 1 hour.
13) The obtained mixture was drained and then rinsed three times with DMF and three times with methanol, with nitrogen sparging for 30 seconds each time, and drained and dried.

**Table 5. Addition order**

| # | **Reaction order** | **Reaction reagent** |
|---|---|---|
| 1 | Fmoc-Asp(OAll)-OH (1.0 eq.) | DIEA (4.0 eq.) |
| 2 | Fmoc-hArg(Pbf)-OH (3.0 eq.) | HBTU (2.85 eq.), DIEA (6.0 eq.) |
| 3 | Fmoc-Lys(Alloc)-OH (3.0 eq.) | HATU (2.85 eq.), DIEA (6.0 eq.) |

### Cleavage and purification:

5) A cleavage buffer (20% HFIP/DCM) was added to a flask containing the side chain-protected polypeptide and stirred at room temperature for 30 minutes × 2 times. The solution was collected, spun to dry, and separated by reverse phase preparation to give intermediate **3**.

**Table 6. Purification conditions**

| Separation conditions | |
|---|---|
| Dissolution condition | Dissolved in 20% CH₃CN/H₂O |
| Machine model | SHIMADZU LC-8A Waters |
| Mobile phase | A: H₂O (containing 0.075% TFA) |
| | B CH₃CN |
| Separation gradient | 30-70%-60 min, retention time: 22 min |
| Column type | Welch Xtimate^{®}C18, 250*50 mm, 10 µm, 120Å |
| Flow rate | 80 mL/min |
| Wavelength | 214/254 nm |
| Temperature | 25°C |

### 2. Synthesis of intermediate 4

### Polypeptide synthesis:

The polypeptide was synthesized using the standard step-wise synthesis method.
1) DMF (20 mL) was added to a container containing Rink amide MBHA resin (0.5 mmol, 1.85 g), and the resin was allowed to swell for 2 hours.
2) The obtained mixture was drained and then rinsed three times with DMF, with nitrogen sparging for 30 seconds each time.
3) A 20% piperidine/DMF was added, and then the system reacted for 30 minutes.
4) The obtained mixture was drained and then rinsed five times with DMF, with nitrogen sparging for 30 seconds each time.
5) A Fmoc-protected amino acid solution was added, and the system was sparged with nitrogen for 30 seconds. A condensing agent was then added, and the system reacted under N₂ sparging for approximately 1 hour.
6) Steps 2 to 5 were repeated to condense the next amino acid.

**Table 7. Addition order**

| # | **Raw material** | **Condensing agent** |
|---|---|---|
| 1 | Fmoc-Cys(Trt)-OH (3.0 eq.) | HATU (2.85 eq.), DIEA (6.0 eq.) |
| 2 | Fmoc-hArg(Pbf)-OH (3.0 eq.) | HATU (2.85 eq.), DIEA (6.0 eq.) |
| 3 | Fmoc-Trp(Boc)-OH (3.0 eq.) | HATU (2.85 eq.), DIEA (6.0 eq.) |
| 4 | Fmoc-dAsp(OtBu)-OH (3.0 eq.) | HATU (2.85 eq.), DIEA (6.0 eq.) |
| 5 | Fmoc-Pro-OH (3.0 eq.) | HATU (2.85 eq.), DIEA (6.0 eq.) |
| 6 | Fmoc-His(Trt)-OH (3.0 eq.) | HATU (2.85 eq.), DIEA (6.0 eq.) |
| 7 | Fmoc-Leu-OH (3.0 eq.) | HATU (2.85 eq.), DIEA (6.0 eq.) |
| 8 | Fmoc-Cys(Trt)-OH (3.0 eq.) | HATU (2.85 eq.), DIEA (6.0 eq.) |
| 8 | Fmoc-Leu-OH (3.0 eq.) | HATU (2.85 eq.), DIEA (6.0 eq.) |
| 9 | Fmoc-Pro-OH (3.0 eq.) | HATU (2.85 eq.), DIEA (6.0 eq.) |
| 10 | Fmoc-Asn(Trt)-OH (3.0 eq.) | HATU (2.85 eq.), DIEA (6.0 eq.) |
| 11 | Fmoc-Val-OH (3.0 eq.) | HATU (2.85 eq.), DIEA (6.0 eq.) |
| 12 | Fmoc-Leu-OH (3.0 eq.) | HATU (2.85 eq.), DIEA (6.0 eq.) |
| 14 | Fmoc-Hyp(tBu)-OH (3.0 eq.) | HATU (2.85 eq.), DIEA (6.0 eq.) |
| 15 | Fmoc-Cys(Trt)-OH (3.0 eq.) | HATU (2.85 eq.), DIEA (6.0 eq.) |
| 16 | Compound 3 (2.0 eq.) | HATU (1.90 eq.), DIEA (4.0 eq.) |
| 17 | Fmoc-Sar-OH (5.0 eq.) | HATU (4.75 eq.), DIEA (10.0 eq.) |
| 18 | Fmoc-Sar-OH (5.0 eq.) | HATU (4.75 eq.), DIEA (10.0 eq.) |
| 19 | Fmoc-Sar-OH (5.0 eq.) | HATU (4.75 eq.), DIEA (10.0 eq.) |
| 20 | Fmoc-Sar-OH (5.0 eq.) | HATU (4.75 eq.), DIEA (10.0 eq.) |
| 21 | Fmoc-Sar-OH (5.0 eq.) | HATU (4.75 eq.), DIEA (10.0 eq.) |
| 22 | Fmoc-Sar-OH (5.0 eq.) | HATU (4.75 eq.), DIEA (10.0 eq.) |
| 23 | Fmoc-Sar-OH (5.0 eq.) | HATU (4.75 eq.), DIEA (10.0 eq.) |
| 24 | Fmoc-Sar-OH (5.0 eq.) | HATU (4.75 eq.), DIEA (10.0 eq.) |
| 25 | Fmoc-Sar-OH (5.0 eq.) | HATU (4.75 eq.), DIEA (10.0 eq.) |
| 26 | Fmoc-Sar-OH (5.0 eq.) | HATU (4.75 eq.), DIEA (10.0 eq.) |
| 27 | Fmoc-β-Ala-OH (3.0 eq.) | HATU (2.85 eq.), DIEA (6.0 eq.) |

### Polypeptide cleavage and purification:

1) A cleavage buffer (90% TFA/2.5% TIS/2.5% H₂O/5.0% DTT) was added to a flask containing the side chain-protected polypeptide and stirred at room temperature for 2 hours.
2) The polypeptide was sedimented with ice isopropyl ether and centrifuged using a centrifuge (3000 rpm, 3 mins).
3) The obtained polypeptide was washed twice more with isopropyl ether.
4) The crude polypeptide was dried to give the TFA salt of intermediate 4 (crude product).

### 3. Synthesis of a TFA salt of intermediate 5

The crude TFA salt of intermediate 4 (1.40 g, 470 µmοl) was dissolved in 50% MeCN/H₂O (500 mL), and TATA (140 mg, 560 µmοl) was slowly added to the stirred solution at room temperature. The reaction mixture was stirred at room temperature for 30 minutes, and then the pH was adjusted to 8 with NH₄HCO₃. The reaction was stirred at room temperature for an additional 12 hours. When LCMS showed that the reaction was complete, stirring was stopped. The mixture was purified by reverse phase preparation (A: 0.075% TFA/ H₂O, B: CH₃CN) to give the TFA salt of intermediate **5** (crude product).

**Table 8. Purification conditions**

| Separation conditions | |
|---|---|
| Dissolution condition | Dissolved in 20% MeCN/H₂O |
| Machine model | SHIMADZU LC-8A Waters |
| Mobile phase | A: H₂O (0.075% TFA) |
| | B CH₃CN |
| Separation gradient | 14-34%-60 min, retention time: 20 min |
| Column type | Welch Xtimate^{®}C18, 250*50 mm, 10 µm, 120Å |
| Flow rate | 80 mL/min |
| Wavelength | 214/254 nm |
| Temperature | 25°C |

### 4. Synthesis of an acetate salt of compound PDC 4

The TFA salt of intermediate 5 (60.0 mg, 18.6 µmοl) was dissolved in DMF (0.3 mL), and then DIEA (12.9 µL, 74.4 µmοl) was added and stirred at room temperature for 10 minutes. Next, the TFA salt of compound **INT_1** (24.8 mg, 18.6 µmοl) was dissolved in DMF (0.2 mL), dropwise added to the above reaction solution of intermediate 5, and then stirred at room temperature for 2 hours. The reaction solution was filtered to remove insoluble residues. The filtrate was directly purified by reverse phase preparation (TFA system), lyophilized, and then converted to an acetate salt by preparation to give the acetate salt of compound **PDC_4.**

### Example 5

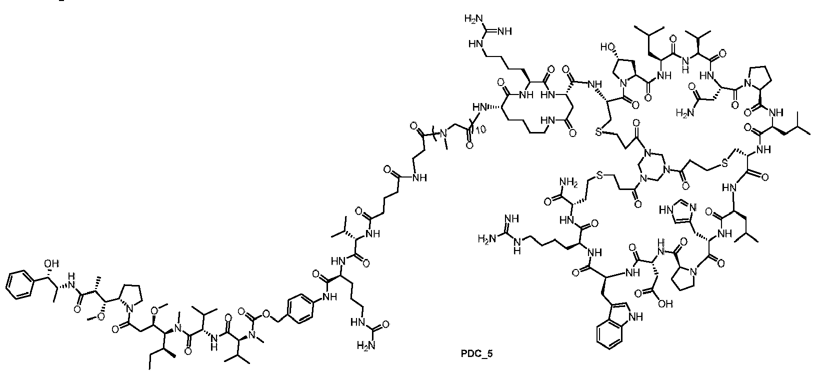

### Synthetic route:

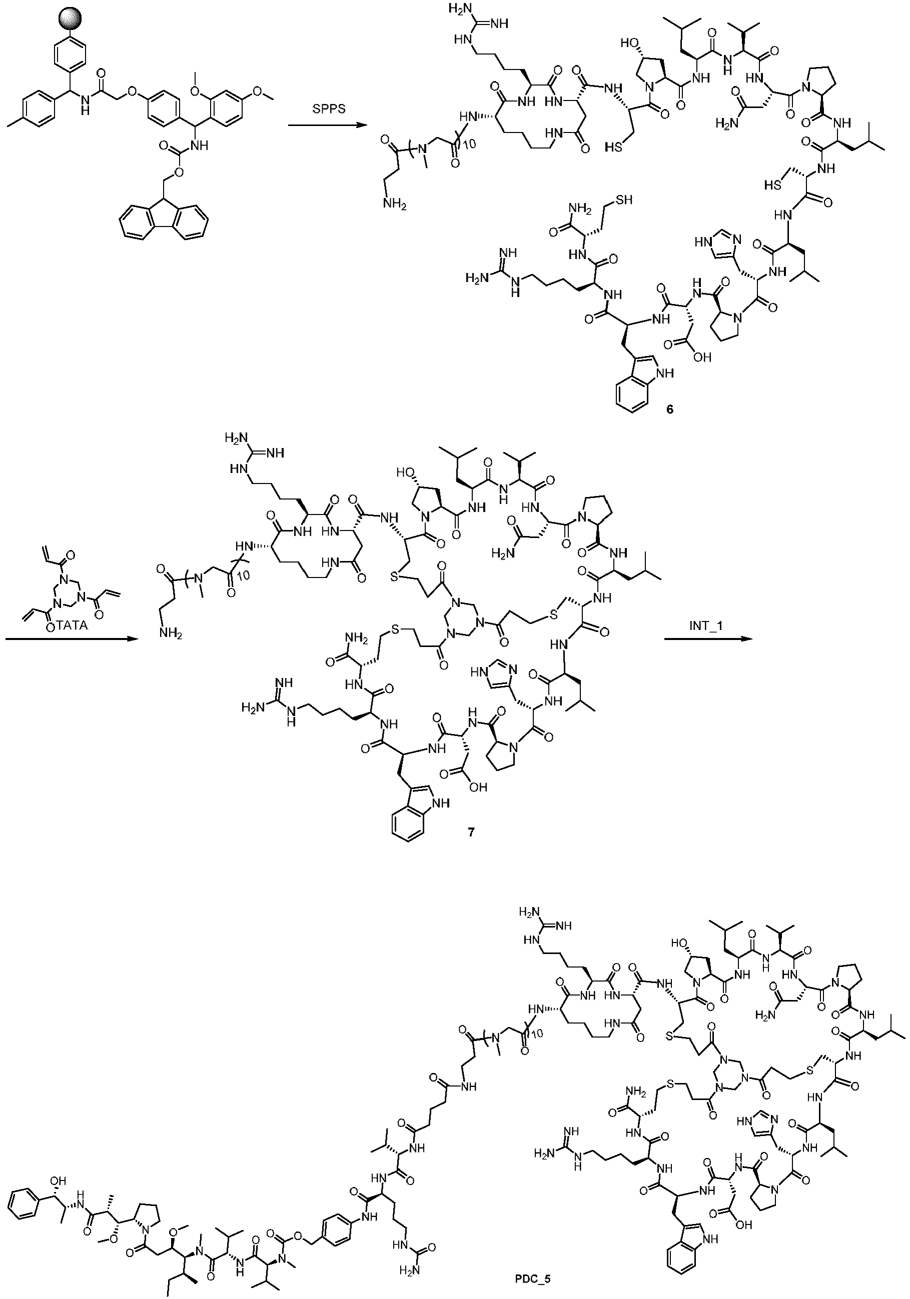

### Synthesis of intermediate 6:

The polypeptide was synthesized using the standard step-wise synthesis method.
1) DMF (30 mL) was added to a container containing Rink amide MBHA resin (0.5 mmol, 1.8 g, with 0.28 mmol/g of a substrate), and the resin was allowed to swell for 2 hours.
2) The obtained mixture was drained and then rinsed three times with DMF, with nitrogen sparging for 30 seconds each time.
3) A 20% piperidine/DMF was added, and then the system reacted for 30 minutes.
4) The obtained mixture was drained and then rinsed five times with DMF, with nitrogen sparging for 30 seconds each time.
5) A Fmoc-protected amino acid solution was added for 30 seconds, followed by a condensing agent, and the system reacted under N₂ sparging for approximately 1 hour.
6) Steps 2 to 5 were repeated to condense the next amino acid.

**Table 9. Addition order**

| # | **Raw material** | **Condensing agent** |
|---|---|---|
| 1 | Fmoc-hCys(Trt)-OH (2.0 eq.) | HATU (1.90 eq.), DIEA (4.0 eq.) |
| 2 | Fmoc-hArg(Pbf)-OH (2.0 eq.) | HATU (1.90 eq.), DIEA (4.0 eq.) |
| 3 | Fmoc-Trp(Boc)-OH (3.0 eq.) | HATU (2.85 eq.), DIEA (6.0 eq.) |
| 4 | Fmoc-d-Asp(OtBu)-OH (3.0 eq.) | HATU (2.85 eq.), DIEA (6.0 eq.) |
| 5 | Fmoc-Pro-OH (3.0 eq.) | HATU (2.85 eq.), DIEA (6.0 eq.) |
| 6 | Fmoc-His(Trt)-OH (3.0 eq.) | HATU (2.85 eq.), DIEA (6.0 eq.) |
| 7 | Fmoc-Leu-OH (3.0 eq.) | HATU (2.85 eq.), DIEA (6.0 eq.) |
| 8 | Fmoc-Cys(Trt)-OH (3.0 eq.) | HATU (2.85 eq.), DIEA (6.0 eq.) |
| 9 | Fmoc-Leu-OH (3.0 eq.) | HATU (2.85 eq.), DIEA (6.0 eq.) |
| 10 | Fmoc-Pro-OH (3.0 eq.) | HATU (2.85 eq.), DIEA (6.0 eq.) |
| 11 | Fmoc-Asn(Trt)-OH (3.0 eq.) | HATU (2.85 eq.), DIEA (6.0 eq.) |
| 12 | Fmoc-Val-OH (3.0 eq.) | HATU (2.85 eq.), DIEA (6.0 eq.) |
| 13 | Fmoc-Leu-OH (3.0 eq.) | HATU (2.85 eq.), DIEA (6.0 eq.) |
| 14 | Fmoc-Hyp-OH (3.0 eq.) | HATU (2.85 eq.), DIEA (6.0 eq.) |
| 15 | Fmoc-Cys(Trt)-OH (3.0 eq.) | HATU (2.85 eq.), DIEA (6.0 eq.) |
| 16 | Fmoc-Asp(OAll)-OH (2.0 eq.) | HATU (1.90 eq.), DIEA (4.0 eq.) |
| 17 | Fmoc-HArg(Pbf)-OH (2.0 eq.) | HATU (1.90 eq.), DIEA (4.0 eq.) |
| 18 | Fmoc-Lys(Alloc)-OH (2.0 eq.) | HATU (1.90 eq.), DIEA (4.0 eq.) |
| - | Pd(PPh₃)₄ (0.1 *eq.*), PhSiH₃ (10.0 *eq.*) | 15 mins, 3 times |
| - | - | HATU (4.75 eq.), DIEA (10.0 eq.) |
| 19 | Fmoc-Sar-OH (5.0 eq.) | HATU (4.75 eq.), DIEA (10.0 eq.) |
| 20 | Fmoc-Sar-OH (5.0 eq.) | HATU (4.75 eq.), DIEA (10.0 eq.) |
| 21 | Fmoc-Sar-OH (5.0 eq.) | HATU (4.75 eq.), DIEA (10.0 eq.) |
| 22 | Fmoc-Sar-OH (5.0 eq.) | HATU (4.75 eq.), DIEA (10.0 eq.) |
| 23 | Fmoc-Sar-OH (5.0 eq.) | HATU (4.75 eq.), DIEA (10.0 eq.) |
| 24 | Fmoc-Sar-OH (5.0 eq.) | HATU (4.75 eq.), DIEA (10.0 eq.) |
| 25 | Fmoc-Sar-OH (5.0 eq.) | HATU (4.75 eq.), DIEA (10.0 eq.) |
| 26 | Fmoc-Sar-OH (5.0 eq.) | HATU (4.75 eq.), DIEA (10.0 eq.) |
| 27 | Fmoc-Sar-OH (5.0 eq.) | HATU (4.75 eq.), DIEA (10.0 eq.) |
| 28 | Fmoc-Sar-OH (5.0 eq.) | HATU (4.75 eq.), DIEA (10.0 eq.) |
| 29 | Fmoc-β-Ala-OH (3.0 eq.) | HATU (2.85 eq.), DIEA (6.0 eq.) |

The Fmoc protecting groups were removed with 20% piperidine/DMF for 30 minutes. The condensation was detected using a chromogenic reaction. The resin was rinsed 3-5 times with DMF after each reaction.

### Polypeptide cleavage and purification:

1) A cleavage buffer (90% TFA/2.5% TIS/2.5% H₂O/5.0% DTT) was added to a flask containing the side chain-protected polypeptide and stirred at room temperature for 2 hours.
2) The polypeptide was sedimented with ice isopropyl ether and centrifuged using a centrifuge (3000 rpm, 3 mins).
3) The obtained polypeptide was washed twice more with isopropyl ether.
4) The crude product was dried to give intermediate **6**.

### Synthesis of a TFA salt of intermediate 7:

The crude polypeptide intermediate **6** (1.20 g, 418 µmοl) was dissolved in 50% MeCN/H₂O (500 mL), and TATA (150 mg, 600 µmοl) was slowly added to the stirred solution at room temperature. The reaction mixture was stirred at room temperature for 30 minutes, and then the pH was adjusted to 8 with NH₄HCO₃. The reaction was stirred at room temperature for an additional 12 hours. When LCMS showed that the reaction was complete, stirring was stopped. The mixture was purified by reverse phase preparation (TFA system) to give the TFA salt of intermediate **7**.

**Table 10. Purification conditions**

| Separation conditions | |
|---|---|
| Dissolution condition | Dissolved in 20% MeCN/H₂O |
| Machine model | SHIMADZU LC-8A Waters |
| Mobile phase | A: H₂O (containing 0.075% TFA) |
| | B CH₃CN |
| Separation gradient | 20-40%-60 min, retention time: 18 min |
| Column type | Welch Ultimate^{®} XB-C18, 250*50 mm, 10 µm, 120Å + Welch Xtimate^{®}C18, 250*50 mm, 10 µm, 120Å |
| Flow rate | 80 mL/min |
| Wavelength | 214/254 nm |
| Temperature | 25°C |

### Synthesis of an acetate salt of PDC_5:

The TFA salt of polypeptide intermediate **7** (60.6 mg) and the TFA salt of compound **INT_1** (25.0 mg) were dissolved in DMF (0.7 mL), and DIEA (9.68 mg, 74.9 µmol, 13.0 µL) was added. The obtained mixture was stirred at room temperature for 2 hours. When LC-MS showed that the reaction was complete, the reaction was stopped. The reaction solution was filtered to remove insoluble residues. The filtrate was directly purified by reverse phase preparation (TFA system), lyophilized, and then converted to an acetate salt by preparation to give the acetate salt of **PDC_5**.

### Example 6

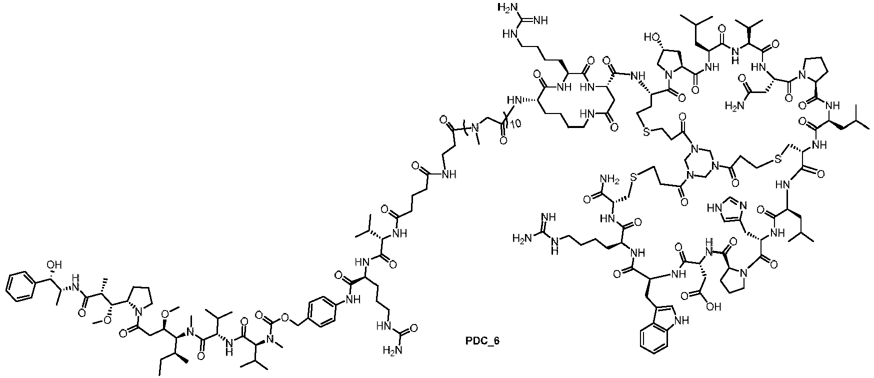

An acetate salt of **PDC_6** was synthesized by reference to the synthesis of Example 5, where raw material 1 in the table 9 addition order of Example 5 was replaced with Fmoc-Cys(Trt)-OH (2.0 eq.) and raw material 15 was replaced with Fmoc-hCys(Trt)-OH (3.0 eq.).

### Example 7

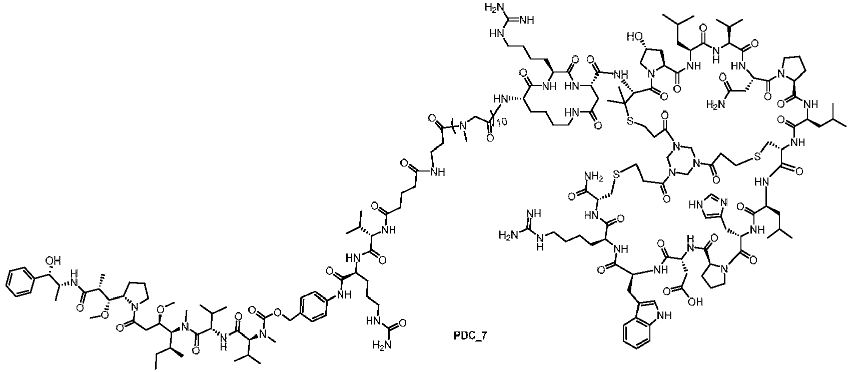

### Synthetic route:

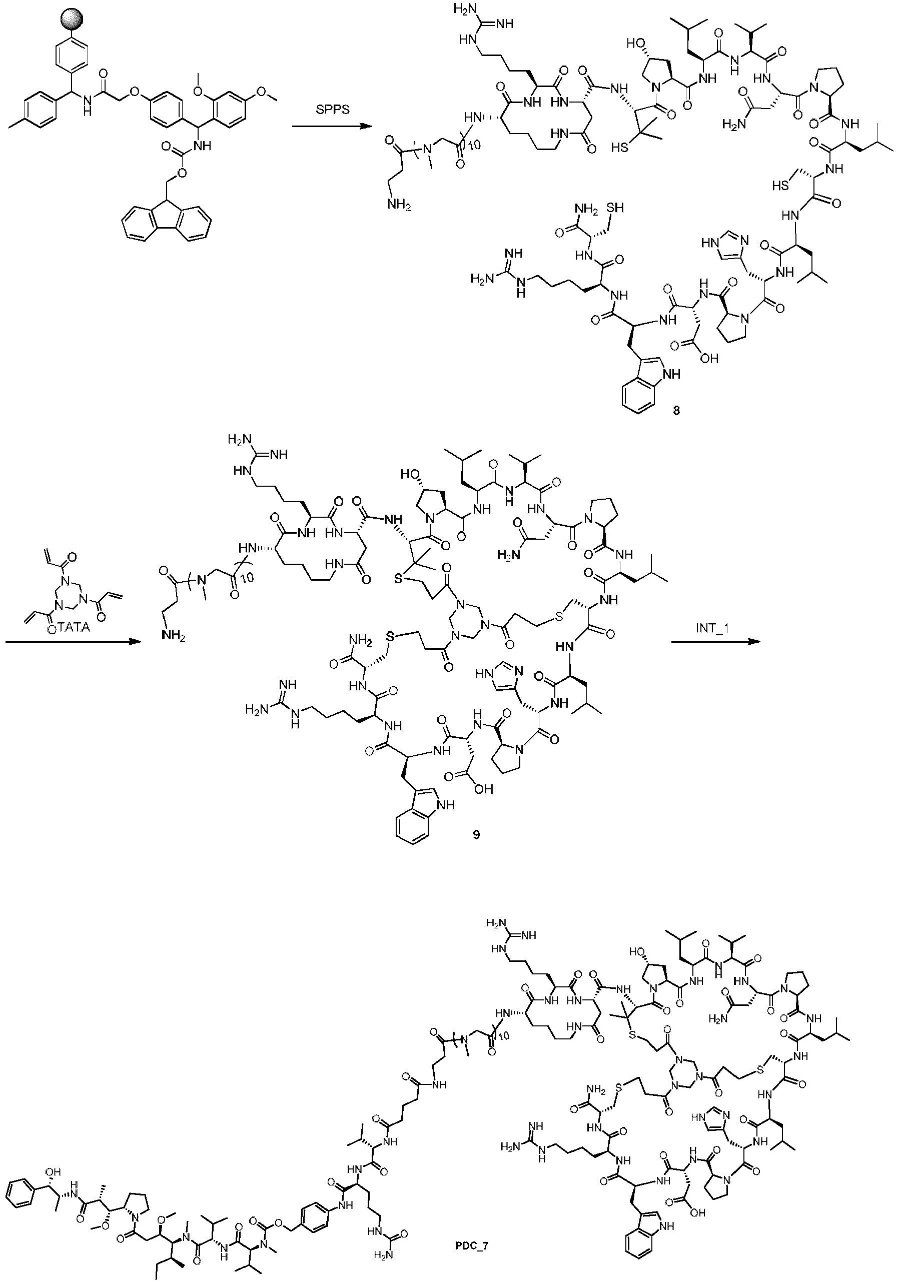

### Synthesis of intermediate 8:

The polypeptide was synthesized using the standard step-wise synthesis method.
1) DMF (30 mL) was added to a container containing Rink amide MBHA resin (0.5 mmol, 1.5 g, with 0.33 mmol/g of a substrate), and the resin was allowed to swell for 2 hours.
2) The obtained mixture was drained and then rinsed three times with DMF, with nitrogen sparging for 30 seconds each time.
3) A 20% piperidine/DMF was added, and then the system reacted for 30 minutes.
4) The obtained mixture was drained and then rinsed five times with DMF, with nitrogen sparging for 30 seconds each time.
5) A Fmoc-protected amino acid solution was added for 30 seconds, followed by a condensing agent, and the system reacted under N₂ sparging for approximately 1 hour.
6) Steps 2 to 5 were repeated to condense the next amino acid.

**Table 11. Addition order**

| # | **Raw material** | **Condensing agent** |
|---|---|---|
| 1 | Fmoc-Cys(Trt)-OH (2.0 eq.) | HATU (2.85 eq.), DIEA (4.0 eq.) |
| 2 | Fmoc-hArg(Pbf)-OH (2.0 eq.) | HATU (1.90 eq.), DIEA (4.0 eq.) |
| 3 | Fmoc-Trp(Boc)-OH (3.0 eq.) | HATU (2.85 eq.), DIEA (6.0 eq.) |
| 4 | Fmoc-d-Asp(OtBu)-OH (3.0 eq.) | HATU (2.85 eq.), DIEA (6.0 eq.) |
| 5 | Fmoc-Pro-OH (3.0 eq.) | HATU (2.85 eq.), DIEA (6.0 eq.) |
| 6 | Fmoc-His(Trt)-OH (3.0 eq.) | HATU (2.85 eq.), DIEA (6.0 eq.) |
| 7 | Fmoc-Leu-OH (3.0 eq.) | HATU (2.85 eq.), DIEA (6.0 eq.) |
| 8 | Fmoc-Cys(Trt)-OH (3.0 eq.) | HATU (2.85 eq.), DIEA (6.0 eq.) |
| 9 | Fmoc-Leu-OH (3.0 eq.) | HATU (2.85 eq.), DIEA (6.0 eq.) |
| 10 | Fmoc-Pro-OH (3.0 eq.) | HATU (2.85 eq.), DIEA (6.0 eq.) |
| 11 | Fmoc-Asn(Trt)-OH (3.0 eq.) | HATU (2.85 eq.), DIEA (6.0 eq.) |
| 12 | Fmoc-Val-OH (3.0 eq.) | HATU (2.85 eq.), DIEA (6.0 eq.) |
| 13 | Fmoc-Leu-OH (3.0 eq.) | HATU (2.85 eq.), DIEA (6.0 eq.) |
| 14 | Fmoc-Hyp-OH (3.0 eq.) | HATU (2.85 eq.), DIEA (6.0 eq.) |
| 15 | Fmoc-Pen(Trt)-OH (3.0 eq.) | HATU (2.85 eq.), DIEA (6.0 eq.) |
| 16 | Fmoc-Asp(OAll)-OH (2.0 eq.) | HATU (1.90 eq.), DIEA (4.0 eq.) |
| 17 | Fmoc-HArg(Pbf)-OH (2.0 eq.) | HATU (1.90 eq.), DIEA (4.0 eq.) |
| 18 | Fmoc-Lys(Alloc)-OH (2.0 eq.) | HATU (1.90 eq.), DIEA (4.0 eq.) |
| - | Pd(PPh₃)₄ (0.1 *eq.*), PhSiH₃ (10.0 *eq.*) | 15 mins, 3 times |
| - | - | HATU (4.75 eq.), DIEA (10.0 eq.) |
| 19 | Fmoc-Sar-OH (5.0 eq.) | HATU (4.75 eq.), DIEA (10.0 eq.) |
| 20 | Fmoc-Sar-OH (5.0 eq.) | HATU (4.75 eq.), DIEA (10.0 eq.) |
| 21 | Fmoc-Sar-OH (5.0 eq.) | HATU (4.75 eq.), DIEA (10.0 eq.) |
| 22 | Fmoc-Sar-OH (5.0 eq.) | HATU (4.75 eq.), DIEA (10.0 eq.) |
| 23 | Fmoc-Sar-OH (5.0 eq.) | HATU (4.75 eq.), DIEA (10.0 eq.) |
| 24 | Fmoc-Sar-OH (5.0 eq.) | HATU (4.75 eq.), DIEA (10.0 eq.) |
| 25 | Fmoc-Sar-OH (5.0 eq.) | HATU (4.75 eq.), DIEA (10.0 eq.) |
| 26 | Fmoc-Sar-OH (5.0 eq.) | HATU (4.75 eq.), DIEA (10.0 eq.) |
| 27 | Fmoc-Sar-OH (5.0 eq.) | HATU (4.75 eq.), DIEA (10.0 eq.) |
| 28 | Fmoc-Sar-OH (5.0 eq.) | HATU (4.75 eq.), DIEA (10.0 eq.) |
| 29 | Fmoc-β-Ala-OH (3.0 eq.) | HATU (2.85 eq.), DIEA (6.0 eq.) |

The Fmoc protecting groups were removed with 20% piperidine/DMF for 30 minutes. The condensation was detected using a chromogenic reaction. The resin was rinsed 3-5 times with DMF after each reaction.

### Polypeptide cleavage and purification:

1) A cleavage buffer (90% TFA/2.5% TIS/2.5% H₂O/5.0% DTT) was added to a flask containing the side chain-protected polypeptide and stirred at room temperature for 2 hours.
2) The polypeptide was sedimented with ice isopropyl ether and centrifuged using a centrifuge (3000 rpm, 3 mins).
3) The obtained polypeptide was washed twice more with isopropyl ether.
4) The crude product was dried to give intermediate **8**.

### Synthesis of a TFA salt of intermediate 9:

The crude polypeptide intermediate **8** (1.50 g, 500 µmοl) was dissolved in 50% MeCN/H₂O (500 mL), and TATA (186 mg, 750 µmοl) was slowly added to the stirred solution at room temperature. The reaction mixture was stirred at room temperature for 30 minutes, and then the pH was adjusted to 8 with NH₄HCO₃. The reaction was stirred at room temperature for an additional 12 hours. When LCMS showed that the reaction was complete, stirring was stopped. The mixture was purified by reverse phase preparation (TFA system) to give the TFA salt of intermediate **9**.

**Table 12. Purification conditions**

| Separation conditions | |
|---|---|
| Dissolution condition | Dissolved in 20% MeCN/H₂O |
| Machine model | SHIMADZU LC-8A Waters |
| Mobile phase | A: H₂O (containing 0.075% TFA) |
| | B CH₃CN |
| Separation gradient | 20-40%-60 min, retention time: 18 min |
| Column type | Welch Ultimate^{®} XB-C18, 250*50 mm, 10 µm, 120Å + Welch |
| | Xtimate^{®}C18, 250*50 mm, 10 µm, 120Å |
| Flow rate | 80 mL/min |
| Wavelength | 214/254 nm |
| Temperature | 25°C |

### Synthesis of an acetate salt of PDC_7:

The TFA salt of polypeptide intermediate **9** (40.0 mg) and the TFA salt of compound **INT_1** (17.2 mg) were dissolved in DMF (0.5 mL), and DIEA (6.36 mg, 49.2 µmοl) was added. The obtained mixture was stirred at room temperature for 5 hours. When LC-MS showed that the reaction was complete, the reaction was stopped. The reaction solution was filtered to remove insoluble residues. The filtrate was directly purified by reverse phase preparation (TFA system), lyophilized, and then converted to an acetate salt by preparation to give the acetate salt of **PDC_7.**

### Example 8

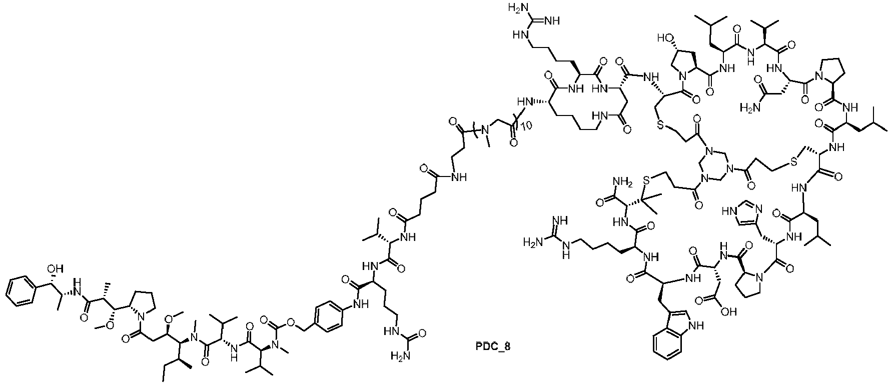

An acetate salt of **PDC_8** was synthesized by reference to the synthesis of Example 7, where raw material 1 in the table 11 addition order of Example 7 was replaced with Fmoc-Pen(Trt)-OH (2.85 eq.) and raw material 15 was replaced with Fmoc-Cys(Trt)-OH (2.85 eq.).

**Table 13. Structural sequences and mass spectrometry data of PDC_4 to 8**

| **Comp. No.** | **PDC structure** | **Mass (Found)** | **Molecular Weight (M)** |
|---|---|---|---|
| **PDC_4** | MMAE-PABC-Cit-Val-Glutaryl-β-Ala-Sar10-Lys-hArg-Asp-Cys-Hyp-Leu- Val-Asn-Pro-Leu-Cys-Leu-His-Pro-dAsp-Trp-hArg-Cys-NH2,TATA | 1111.3 (M+4H⁺)/4 | 4441.2 |
| **PDC_5** | MMAE-PABC-Cit-Val-Glutaryl-β-Ala-Sar10-Lys-hArg-Asp-Cys-Hyp-Leu- Val-Asn-Pro-Leu-Cys-Leu-His-Pro-dAsp-Trp-hArg-hCys-NH2,TATA | 1486.3 (M+3H⁺)/3 | 4455.9 |
| **PDC_6** | MMAE-PABC-Cit-Val-Glutaryl-β-Ala-Sar10-Lys-hArg-Asp-hCys-Hyp- Leu-Val-Asn-Pro-Leu-Cys-Leu-His-Pro-dAsp-Trp-hArg-Cys-NH2,TATA | 1486.0 (M+3H⁺)/3 | 4455.0 |
| **PDC_7** | MMAE-PABC-Cit-Val-Glutaryl-β-Ala-Sar10-Lys-hArg-Asp-Pen-Hyp-Leu- Val-Asn-Pro-Leu-Cys-Leu-His-Pro-dAsp-Trp-hArg-Cys-NH2,TATA | 1490.8 (M+3H⁺)/3 | 4469.4 |
| **PDC_8** | MMAE-PABC-Cit-Val-Glutaryl-β-Ala-Sar10-Lys-hArg-Asp-Cys-Hyp-Leu- Val-Asn-Pro-Leu-Cys-Leu-His-Pro-dAsp-Trp-hArg-Pen-NH2,TATA | 1118.3 (M+4H⁺)/4 | 4469.2 |

### Bioassay

### Test Example 1. Binding capacity test of the compounds of the present invention to EphA2 protein

### 1. Experimental purpose

To detect the affinity of the compound to be tested to the target protein EphA2 using the SPR method.

### 2. Materials and instruments

- Biacore 8K (GE Healthcare)
- 96-well Plate (Cat# 650101, greiner bio-one)
- CM5 chip (Cat# BR-1005-30, GE Healthcare)
- Amine Coupling Kit (Cat# BR-1000-50, GE Healthcare)
   EDC
   NHS
   1 M ethanolamine
- 10 mM sodium acetate pH 4.5 (Cat# BR-1003-50, GE Healthcare)
- DMSO (Cat# D4540, Sigma)
- P20 (Cat# BR-1000-54, GE Healthcare)
- PBS (Cat# BR-1006-72, GE Healthcare)
- EphA2 (Cat# 13926-H08HD, Sino Biological)

### 3. Experimental protocol

In this experiment, the amino coupling method was used. The target protein EphA2 was directly immobilized on the CM5 chip using Biacore 8K. The compound to be tested as an analyte was then diluted to a required concentration gradient with a buffer (10 mM PBS, pH 7.4, 137 mM NaCl, 2.7 mM KCl, 5% DMSO, and 0.05% P20) for multi-cycle kinetic detection, in which each cycle is formed by 180 seconds of sample injection and 180 seconds of dissociation, and then the next cycle was performed, to obtain kinetic analysis data on the affinity of the target protein EphA2 to the compound to be tested. The final data were subjected to Kinetics fitting analysis based on a 1:1 model using Biacore Insight Evaluation Software (V 2.0.15.12933).

### 4. Experimental method and procedure

1) Preparation of the buffer: 10 mM PBS, pH 7.4, 137 mM NaCl, 2.7 mM KCl, 5% DMSO, and 0.05% P20.
2) Activation of the CM5 chip: The CM5 chip was activated with 400 mM EDC and 100 mM NHS at a flow rate of 10 µL/min for 420 seconds.
3) Coupling of the target protein: The target protein was diluted to 10 µg/mL with 10 mM sodium acetate (pH 4.5) and coupled at a flow rate of 10 µL/min for 284 s. The 1#, 2#, and 3# channels on the chip were used in the experiment, and the coupling results were 1639.9RU, 1747.8RU, and 1702.2RU, respectively.
4) Blocking of the CM5 chip: The CM5 chip was blocked with 1 M ethanolamine at a flow rate of 10 µL/min for 420 seconds.
5) Analyte concentration: The compound to be tested was diluted with the buffer. The compound to be tested was diluted from 100 nM to 0.78 nM in a 2-fold gradient.
6) Sample injection analysis: Each concentration of the working solution of the compound to be tested corresponded to one cycle, with a flow rate of 30 µL/min for binding for 180 seconds and dissociating for 180 seconds. The last cycle was a 5% DMSO solvent correction cycle.
7) All results were subjected to kinetics fitting analysis based on a 1:1 model.

### 5. Experimental results

The experimental data of five effective concentrations of the compounds of the present invention were selected for Kinetics fitting analysis based on a 1:1 model using Biacore Insight Evaluation Software (V 2.0.15.12933). The results are shown in Table 14.

**Table 14. Binding results of the compounds of the present invention to Human EphA2 SPR**

| Compound | Human EphA2 SPR k_{D} (nM) |
|---|---|
| Acetate salt of PDC_2 | 33 |
| Acetate salt of PDC_4 | 13.30 |
| Acetate salt of PDC_5 | 6.73 |
| Acetate salt of PDC_6 | 5.67 |
| Acetate salt of PDC_7 | 8.39 |
| Acetate salt of PDC_8 | 3.46 |

**Conclusion:** The compounds of the present invention have a strong binding effect to EphA2.

### Test Example 2: In vitro anti-proliferative activity of the compounds of the present invention on NCI-H1975 and SK-OV-3 cells

### 1. Experimental purpose

To investigate the effect of the compounds of the present invention on inhibiting cell proliferation by detecting the effect of the compounds on cell activity in vitro in tumor cell lines NCI-H1975 and SK-OV-3.

### 2. Experimental design:

### Cell culture

The tumor cell lines were cultured in a 37°C, no CO₂ incubator and a 37°C, 5% CO₂ incubator according to the culture conditions shown in Table 15, respectively. The cells were periodically passaged, and cells in a logarithmic growth phase were taken for plating.

**Table 15. Cell culture conditions**

| Cell line | Tumor type | Growth characteristics | Cell source | Cat. No. | Culture method |
|---|---|---|---|---|---|
| NCI-H19 75 | Human lung cancer cells | Adherent | ATCC | CRL-59 08 | RPMI 1640 + 10% FBS |
| SK-OV-3 | Human ovarian cancer cells | Adherent | ECAC C | 910910 04 | McCoy's 5a + 10% FBS |

### Cell plating

Cells in a logarithmic growth phase were harvested and centrifuged at 1000 rpm for 3 min at room temperature. The supernatant was discarded, and the cells were resuspended in 5 mL of a culture medium. Then, 20 ul of the cell suspension was pipetted and mixed with trypan blue at 1: 1 for staining for 3 min to detect cell viability and to count viable cells. The cell density was adjusted to 4000 NCI-H1975/well or 5000 SK-OV-3/well. Then, 90 µL of the cell suspension was added to each culture plate well, and a cell-free culture medium was added to blank control wells. The culture plate was incubated overnight in a 37°C, no CO₂, and 100% relative humidity incubator and a 37°C, 5% CO₂, and 100% relative humidity incubator, respectively.

### Preparation of a compound stock plate

Preparation of a 400X compound stock plate: The compound to be tested was gradient diluted with DMSO from the highest to the lowest concentration.

**Table 16. Dilution concentrations of the 400X stock plate**

| | | | | | | | | | |
|---|---|---|---|---|---|---|---|---|---|
| | 1 | 2 | 3 | 4 | 5 | 6 | 7 | 8 | 9 |
| A (µM) | 4000 | 1333.3 | 444.44 | 148.15 | 49.383 | 16.461 | 5.487 | 1.829 | 0.610 |

### Preparation of a 10X compound working solution and compound treatment of cells

Preparation of a 10X compound working solution: 78 µl of a cell culture medium was added to a V-bottomed 96-well plate, and 2 µL of the compound was pipetted from the 400X compound stock plate into the cell culture medium in the 96-well plate. Then, 2 µL of DMSO was added to vehicle control and blank control. After adding the compound or DMSO, the mixture was blown uniformly with a multichannel pipettor. Drug addition: 10 µl of the 10X compound working solution was added to the cell culture plate, as shown in Table 1. Then, 10 µL of a DMSO-cell culture medium mixture was added to vehicle control and blank control. The final concentration of DMSO was 0.25%. The 96-well cell plate was placed back into the incubators for 72 h.

### Cell viability assay by CellTiter-Glo luminescence

The detection was performed according to the instructions of the Promega CellTiter-Glo Luminescent Cell Viability Assay kit (Promega-G7573). The luminescence signal was detected on the EnVision^{®} Multi-mode Plate Reader (EnVision 2104-10).

### Data Analysis

The inhibition rate (IR) of the compound to be tested was calculated using the following formula: IR (%) = (1 - (RLU compound - RLU blank control) / (RLU vehicle control - RLU blank control)) * 100%. The inhibition rates of the compounds at different concentrations were calculated in Excel. Then, the inhibition curves were plotted using GraphPad Prism 6.02 software, and the relevant parameters, including the minimum inhibition rate, the maximum inhibition rate, and IC₅₀, were calculated.

Experimental results are shown in Table 17.

**Table 17. Results of anti-tumor cell roliferation**

| Compound to be tested | Tumor cell line | IC₅₀ (µM) | Minimum inhibition rate (%) | Maximum inhibition rate (%) |
|---|---|---|---|---|
| Acetate salt of PDC_8 | NCI-H1975 | 0.079 | -0.51 | 52.07 |
| Acetate salt of PDC_8 | SK-OV-3 | 0.586 | 5.71 | 58.46 |

| | | | | |
|---|---|---|---|---|
| **Conclusion:** The compound of the present invention has a significant inhibitory effect on the proliferation of cells cultured in vitro in the tumor cell lines NCI-H1975 and SK-OV-3. | | | | |

### Test Example 3: In vivo pharmacodynamic study of the compounds of the present invention in a human prostate cancer PC-3 cell subcutaneous xenograft tumor BALB/c nude mouse model

**Experimental purpose:** To investigate the in vivo efficacy of the compounds of the present invention in a human prostate cancer PC-3 cell subcutaneous xenograft tumor model.

**Cell culture:** Human prostate cancer PC-3 cells (ATCC-CRL-1435) were adherent cultured in vitro in F-12K culture medium supplemented with 10% fetal bovine serum, 100 U/mL penicillin, and 100 µg/mL streptomycin in a 37°C, 5% CO₂ incubator. The cells were routinely digested twice a week with pancreatic enzyme-EDTA for passage. When the cell saturation was 80%-90% and the number met the requirement, the cells were harvested, counted, and inoculated.

**Animals:** BALB/c nude mice, female, 6-8 weeks old, weighing 22-27 grams, supplied by Beijing Vital River Laboratory Animal Technology Co., Ltd. A total of 70 mice were inoculated for efficacy assay, and 40 efficacy mice were enrolled.

**Tumor inoculation:** 0.1 mL (10×10⁶) of PC-3 cells were inoculated subcutaneously into the right back of each mouse. The compound was administered in groups of 8 animals per group (n = 8) when the mean tumor volume reached 150-200 mm³.

**Administration volume:** The administration volume was adjusted based on animal weight (administration volume = 10 µL/g).

**Drug formulation:** The compound was formulated into a 1 mg/mL homogeneous solution using 50 mM acetate buffer (pH = 5) in 10% sucrose as a vehicle and stored in a -80°C refrigerator. The homogeneous solution was diluted to a corresponding concentration for IV (intravenous injection) administration on the day of administration.

**Efficacy study:** The compound was administered in groups when the mean tumor volume reached 150-200 mm³. The dose was 0.25 mg/kg, 0.5 mg/kg, and 1.5 mg/kg. The administration frequency was QW×3 weeks.

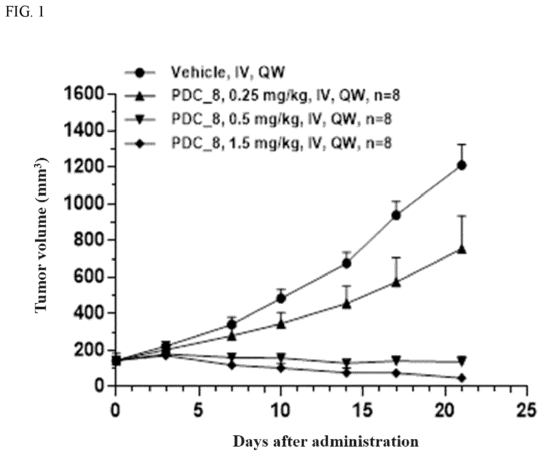

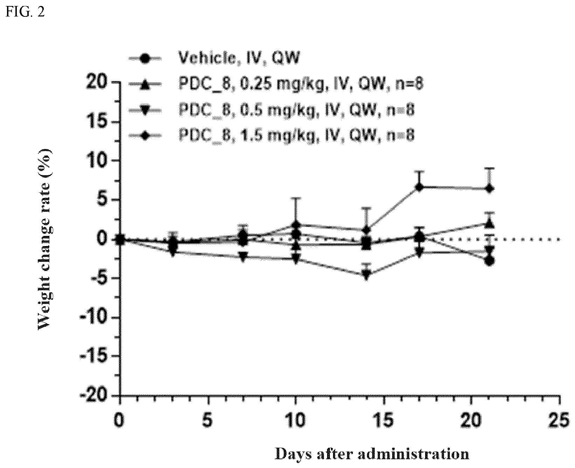

**Experimental results:** See FIG. 1 and FIG. 2.

**Experimental conclusion:** In this assay, 3 dose groups of the compound of the present invention show a significant tumor growth inhibition effect in a human prostate cancer PC-3 cell subcutaneous xenograft tumor model and show a dose-dependence in 0.25 mg/kg, 0.5 mg/kg, and 1.5 mg/kg groups.

### Test Example 4: In vivo pharmacodynamic study of the compounds of the present invention in a human ovarian cancer SK-OV-3 cell subcutaneous xenograft tumor BALB/c nude mouse model

**Experimental purpose:** To evaluate the in vivo efficacy of the compound to be tested in a human ovarian cancer SK-OV-3 cell subcutaneous xenograft tumor model.

**Cell culture:** Human ovarian cancer SK-OV-3 cells (ECACC-91091004) were cultured as monolayers in vitro in McCoy's 5a culture medium supplemented with 10% fetal bovine serum, 100 U/mL penicillin, and 100 µg/mL streptomycin in a 37°C, 5% CO₂ incubator. The cells were routinely digested twice a week with pancreatic enzyme-EDTA for passage. When the cell saturation was 80%-90% and the number met the requirement, the cells were harvested, counted, and inoculated.

**Animals:** BALB/c nude mice, female, 6-8 weeks old, weighing 18-21 grams, supplied by Beijing Vital River Laboratory Animal Technology Co., Ltd.

**Tumor inoculation:** 0.2 mL (10×10⁶) of SK-OV-3 cells were inoculated subcutaneously into the right back of each mouse.

**Administration volume:** The administration volume was adjusted based on animal weight (administration volume = 10 µL/g).

**Drug formulation:** The compound to be tested was formulated into a 1 mg/mL homogeneous solution using 50 mM acetate buffer (pH = 5) in 10% sucrose as a vehicle and stored in a -80°C refrigerator. The homogeneous solution was diluted to a corresponding concentration for IV (intravenous injection) administration on the day of administration.

**Efficacy study:** The compound was administered in groups of 8 animals per group (n = 8) when the mean tumor volume reached 150-200 mm³. The dose was 1 mg/kg and 3 mg/kg. The administration frequency was QW×6 weeks.

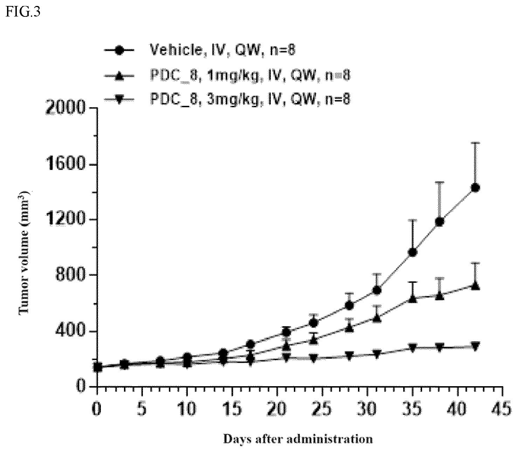

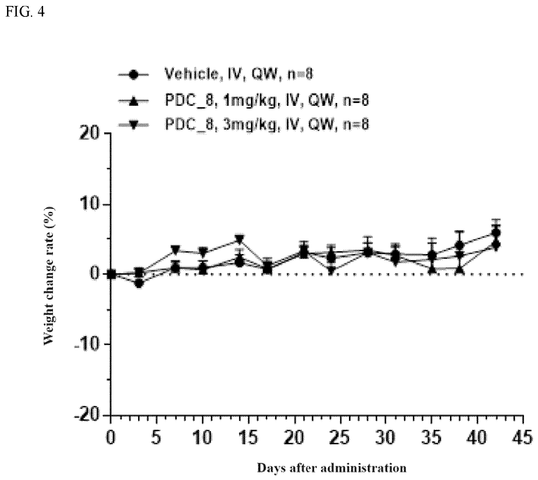

**Experimental results:** See FIG. 3 and FIG. 4.

**Experimental conclusion:** In this assay, 2 dose groups of the compound of the present invention show a significant tumor growth inhibition effect in a human ovarian cancer SK-OV-3 cell subcutaneous xenograft tumor model and show a dose-dependence in 1 mg/kg and 3 mg/kg groups.

**Test Example 5:** In vivo pharmacokinetic analysis of the compounds of the present invention in mice

### A. Experimental purpose

To test the pharmacokinetics of the compounds of the present invention in CD-1 mice

### B. Experimental procedures

The pharmacokinetic characteristics of the compound after intravenous injection and oral administration were tested in CD-1 mice according to a standard protocol. The compound of the present invention was formulated into a clear solution using 50 mM acetate buffer (pH = 5) in 10% sucrose as a vehicle. Two mice were given a single intravenous injection of 2 mg/kg of the compound to be tested. Whole blood was collected at time points of 0.083, 0.25, 0.5, 1, 2, 4, 8, and 24 hours after administration to get plasma. The concentration of the compound to be tested and the concentration of its potential metabolite MMAE were analyzed by the LC-MS/MS method, and the pharmacokinetic parameters, such as maximal concentration (Cₘₐₓ), clearance (Cl), half-life (T_{1/2}), tissue distribution (Vdss), and area under the concentration-time curve (AUC₀₋ₗₐₛₜ), were calculated by Phoenix WinNonlin software.

### C. Experimental results

Experimental results are shown in Table 18.

**Table 18. Pharmacokinetic test results in mice**

| Compound to be tested | **Parameter** | **Value** |
|---|---|---|
| Acetate salt of PDC_8 | Dose (mg/kg) | 2 |
| | C₀ (nM) | 2635 |
| | T_{1/2} (hr) | 0.20 |
| | Vdₛₛ (L/kg) | 0.19 |
| | Cl (mL/min/kg) | 13.0 |
| | AUC₀₋ₗₐₛₜ (nM.h) | 560 |
| MMAE | Cₘₐₓ (nM) | 55 |
| | AUC₀₋ₗₐₛₜ (nM.h) | 51.5 |

| | | |
|---|---|---|
| Conclusion: The compound of the present invention has a short half-life and rapid clearance in CD-1 mice blood, and the release of MMAE in CD-1 mice plasma is extremely low. | | |

### Test Example 6: Pharmacokinetic analysis of the compounds of the present invention in rat plasma

**Experimental purpose:** To test the pharmacokinetics of the compounds of the present invention in SD rats.

**6.1** The pharmacokinetic characteristics of the compound after intravenous injection and oral administration were tested in rats according to a standard protocol. The compound to be tested was formulated into a clear solution using 50 mM acetate buffer (pH = 5) in 10% sucrose as a vehicle. Two SD rats were given a single intravenous injection of 2 mg/kg of the compound to be tested. Whole blood was collected at time points of 0.083, 0.25, 0.5, 1, 2, 4, 8, and 24 hours after administration to get plasma. The concentration of the compound to be tested and the concentration of its potential metabolite MMAE were analyzed by the LC-MS/MS method, and the pharmacokinetic parameters were calculated by Phoenix WinNonlin software.

Experimental results are shown in Table 19.

**Table 19. Pharmacokinetic test results in rats**

| Compound to be tested | **Parameter** | **Value** |
|---|---|---|
| Acetate salt of PDC_8 | Dose (mg/kg) | 2 |
| | C₀ (nM) | 1670 |
| | T_{1/2} (hr) | 0.29 |
| | Vdₛₛ (L/kg) | 0.28 |
| | Cl (mL/min/kg) | 14.4 |
| | AUC₀₋ₗₐₛₜ (nM.h) | 518 |
| MMAE | Cₘₐₓ (nM) | 23.5 |
| | AUC₀₋ₗₐₛₜ (nM.h) | 37.4 |

6.2 The pharmacokinetic characteristics of the compound after intravenous injection and oral administration were tested in rats according to a standard protocol. The compound to be tested was formulated into a clear solution using 50 mM acetate buffer (pH = 5) in 10% sucrose as a vehicle. Three male SD rats were given a single intravenous injection of 2.5 mg/kg, 3.75 mg/kg, or 5 mg/kg of the compound to be tested. Whole blood was collected at time points of 0.083, 0.25, 0.5, 1, 4, and 24 hours after administration to get plasma. The concentration of the compound to be tested and the concentration of its potential metabolite MMAE were analyzed by the LC-MS/MS method, and the pharmacokinetic parameters were calculated.

Experimental results are shown in Table 20.

**Table 20. Pharmacokinetic test results at different doses in rats**

| | | | | |
|---|---|---|---|---|
| Acetate salt of PDC_8 | Dose (mg/kg) | 2.5 | 3.75 | 5 |
| | Cₘₐₓ (nM) | 1955 | 2748 | 4591 |
| | AUC₀₋ₗₐₛₜ (nM.h) | 765 | 3087 | 5654 |
| MMAE | Cₘₐₓ (nM) | 29 | 39 | 52 |
| | AUC₀₋ₗₐₛₜ (nM.h) | 58 | 185 | 435 |

| | | | | |
|---|---|---|---|---|
| **Conclusion:** The compound of the present invention has a short half-life and rapid clearance in rat blood. PDC has an increased exposure with increasing dose and good metabolic stability. The release of MMAE in rat plasma is low and shows a certain dose-dependence. | | | | |

### Test Example 7: Pharmacokinetic analysis of the compounds of the present invention in Cynomolgus macaque plasma

### A. Experimental purpose

To test the pharmacokinetics of the compounds of the present invention in Cynomolgus macaques.

### B. Experimental procedures

The pharmacokinetic characteristics of the compound after intravenous injection and oral administration were tested in Cynomolgus macaques according to a standard protocol. The compound of the present invention was formulated into a clear solution using 50 mM acetate buffer (pH = 5) in 10% sucrose as a vehicle. Two Cynomolgus macaques were given a single intravenous injection of 1 mg/kg of the compound to be tested. Whole blood was collected at time points of 0.083, 0.25, 0.5, 1, 2, 4, 8, and 24 hours after administration to get plasma. The concentration of the compound to be tested and the concentration of its potential metabolite MMAE were analyzed by the LC-MS/MS method, and the pharmacokinetic parameters were calculated by Phoenix WinNonlin software. C. Experimental results are shown in Table 20.

**Table 21. Pharmacokinetic test results in Cynomolgus macaques**

| Compound to be tested | **Parameter** | **Value** |
|---|---|---|
| Acetate salt of PDC_8 | Dose (mg/kg) | 1 |
| | C₀ (nM) | 2473 |
| | T_{1/2} (hr) | 0.31 |
| | Vdₛₛ (L/kg) | 0.12 |
| | Cl (mL/min/kg) | 5.72 |
| | AUC₀₋ₗₐₛₜ (nM.h) | 651 |
| MMAE | Cₘₐₓ (nM) | 5.6 |
| | AUC₀₋ₗₐₛₜ (nM.h) | 28.8 |

| | | |
|---|---|---|
| **Conclusion:** Intravenous injection of the compound of the present invention has a short half-life and rapid clearance in Cynomolgus macaque blood, and the release of MMAE in Cynomolgus macaque plasma is extremely low. | | |

### Test Example 8: Stability analysis of the compounds of the present invention in plasma from different species

### A. Experimental purpose

To test the stability of the compounds of the present invention in plasma from rats, Cynomolgus macaques, and humans.

### B. Experimental procedures

The corresponding incubation plates, including T0, T10, T30, T60, T120, and T240 incubation plates, were added with a 2 µL working solution of the compound to be tested (100 µM). Three parallel wells were prepared for each sample. Then, 98 µL of blank plasma from SD rats, Cynomolgus macaques, and humans was added to the corresponding incubation plates to which the working solution had been added. All samples were incubated in a 37°C water bath. The compound to be tested had a final incubation concentration of 2 µM. At the end of each incubation time point, the corresponding incubation plate was removed, added with stop buffer, precipitated, and centrifuged for 20 minutes. Then, 150 µL of the supernatant was analyzed by the LC-MS/MS method. The concentration of the compound to be tested in the sample was semi-quantitatively determined by the liquid chromatography-tandem mass spectrometry (LC-MS/MS) method.

### C. Experimental results

Experimental results are shown in Table 21.

**Table 22. Stability results of the compound of the present invention in plasma from SD rats, Cynomolgus macaques, and humans**

| Compound No. | Species | T_{1/2} (min) |
|---|---|---|
| Acetate salt of PDC_8 | SD rats | > 578.1 |
| Acetate salt of PDC_8 | Cynomolgus macaques | > 578.1 |
| Acetate salt of PDC_8 | Humans | > 578.1 |

The experimental results obtained using the above method but extending the incubation time of the compound to be tested with human plasma to 72 hours are shown in Table 22.

**Table 23. Stability of the compound of the present invention incubated in human plasma for 72 hours**

| Test time point | PDC_8 remaining% | MMAE generation% |
|---|---|---|
| 0 | 100.0 | 0.1 |
| 0.5 | 96.3 | 0.2 |
| 1 | 103.2 | 0.5 |
| 2 | 86.6 | 0.5 |
| 4 | 100.8 | 0.6 |
| 24 | 89.9 | 0.6 |
| 48 | 96.0 | 0.0 |
| 72 | 78.0 | 3.2 |

| | | |
|---|---|---|
| Conclusion: The compound of the present invention has excellent stability in plasma from three species: SD rats, Cynomolgus macaques, and humans. | | |

### Test Example 9: Stability analysis of the compounds of the present invention in whole blood from different species

### A. Experimental purpose

To test the stability of the compounds of the present invention in whole blood from SD rats and Cynomolgus macaques.

### B. Experimental procedures

The corresponding incubation plates, including T0, T10, T30, T60, T120, and T240 incubation plates, were added with a 2 µL working solution of the compound to be tested (100 µM). Three parallel wells were prepared for each sample. Then, 98 µL of blank whole blood from SD rats and Cynomolgus macaques was added to the corresponding incubation plates to which the working solution had been added. All samples were incubated in a 37°C water bath. The compound to be tested had a final incubation concentration of 2 µM. At the end of each incubation time point, the corresponding incubation plate was removed, added with stop buffer, precipitated, and centrifuged for 20 minutes. Then, 100 µL of the supernatant was diluted with 300 µL of ultrapure water, mixed uniformly, and then analyzed by the LC-MS/MS method. The concentration of the compound to be tested in the sample was semi-quantitatively determined by the liquid chromatography-tandem mass spectrometry (LC-MS/MS) method.

### C. Experimental results

Experimental results are shown in Table 23.

**Table 24. Stability results of the compound of the present invention in whole blood from SD rats and Cynomolgus macaques**

| Compound No. | Species | T_{1/2} (min) |
|---|---|---|
| Acetate salt of PDC_8 | SD rats | 558.6 |
| Acetate salt of PDC_8 | Cynomolgus macaques | > 578.1 |

| | | |
|---|---|---|
| Conclusion: The compound of the present invention has excellent stability in plasma from SD rats and Cynomolgus macaques. | | |

### Test Example 10: Metabolic stability of the compounds of the present invention in hepatocytes from Cynomolgus macaques and humans

### A. Experimental purpose

To investigate the metabolic stability of the compounds of the present invention in hepatocytes from Cynomolgus macaques and humans.

### B. Experimental procedures

Incubations were performed in 96-well plates using an external extraction method. Several 96-well sample precipitation plates, designated T0, T15, T30, T60, T90, T0-MC, T90-MC, and blank matrix, were prepared, respectively. The recovery culture medium and incubation culture medium were removed in advance and placed in a 37°C water bath for preheating. Frozen hepatocytes from Cynomolgus macaques and humans were removed from a liquid nitrogen tank, recovered, and diluted to 0.51×10⁶ cells/mL with the incubation culture medium. Then, 198 µL of hepatocyte suspension (0.5×10⁶ cells/mL) was added to the preheated incubation plates, and 198 µL of the hepatocyte-free incubation culture medium was added to the T0-MC and T90-MC incubation plates as culture medium control groups. All incubation plates were pre-incubated in a 37°C incubator for 10 minutes. Then, a 2 µL working solution of the test sample was added and mixed uniformly. The incubation plates were placed in a shaker inside the incubator for incubation. Three replicates were prepared for each time point. Incubation conditions were 37°C, saturated humidity, and 5% CO₂. In the test system, the test sample had a final concentration of 1 µM; the control had a final concentration of 3 µM; the hepatocytes had a final concentration of 0.5 × 106 cells/mL; and the total organic solvent had a final concentration of 1.0%, in which the DMSO had a final concentration of 0.1%.

At the end of the incubation at the corresponding time point, the incubation plates were removed, from which 25 µL of a compound mixture with the cells was removed and added to sample plates containing 125 µL of stop buffer. For a blank sample plate, 25 µL of the hepatocyte-free incubation culture medium was directly added. All sample plates were sealed with film, shaken at 600 rpm on a plate shaker for 10 minutes, and centrifuged at 3220 ×g for 20 minutes. The test sample supernatant was diluted with purified water at a ratio of 1:3. All samples were mixed uniformly and then analyzed by the LC-MS/MS method.

The concentration of the compound of the present invention in the sample was semi-quantitatively determined by the liquid chromatography-tandem mass spectrometry (LC-MS/MS) method without a standard curve and quality control sample. The ratio of an analyte peak area to an internal standard peak area was used to express the concentration in the sample. Retention times, chromatogram acquisition, and integration of chromatograms of the analyte and the internal standard were processed using the software Analyst (Sciex, Framingham, Massachusetts, USA).

### C. Experimental results

Experimental results are shown in Table 24.

**Table 25. Metabolic stability results of the compound of the present invention in hepatocytes from Cynomolgus macaques and humans**

| Compound No. | Species | T_{1/2} (min) | CL_{int (liver)} (mL/min/kg) |
|---|---|---|---|
| Acetate salt of PDC_8 | Cynomolgus macaques | > 216.8 | < 23 |
| Acetate salt of PDC_8 | Humans | > 216.8 | < 17.8 |

| | | | |
|---|---|---|---|
| **Conclusion:** The compound of the present invention has excellent metabolic stability in liver microsomes from Cynomolgus macaques and humans. | | | |

## Claims

1. A compound represented by formula (V), or a pharmaceutically acceptable salt thereof, 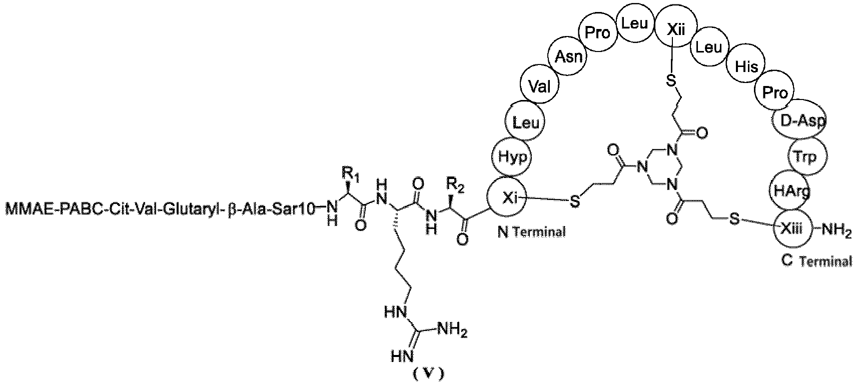 wherein 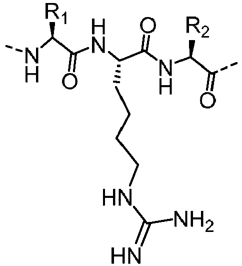 is selected from 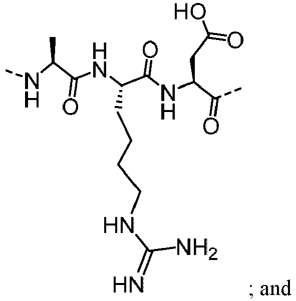
Xi, Xii, and Xiii are each independently selected from Cys, hCys, βCys, Pen,
Xi, Xii, and Xiii being not all Cys;
or 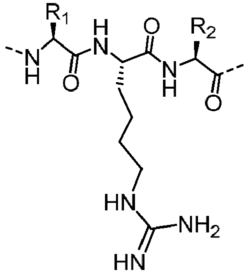 is selected from 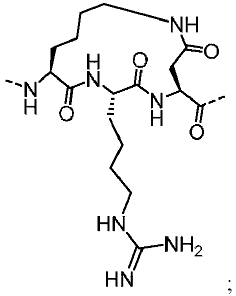
Xi, Xii, and Xiii are each independently selected from Cys, hCys, PCys, and Pen.

2. Compounds represented by the following formulas, or pharmaceutically acceptable salts thereof,

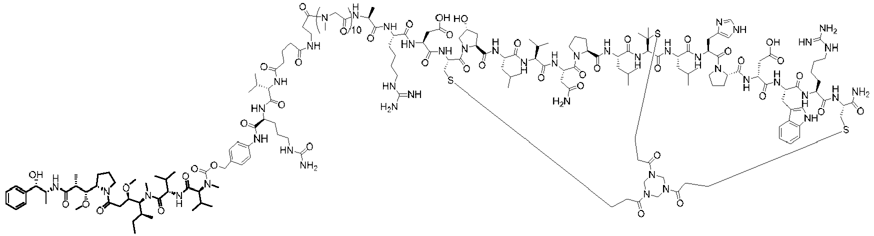

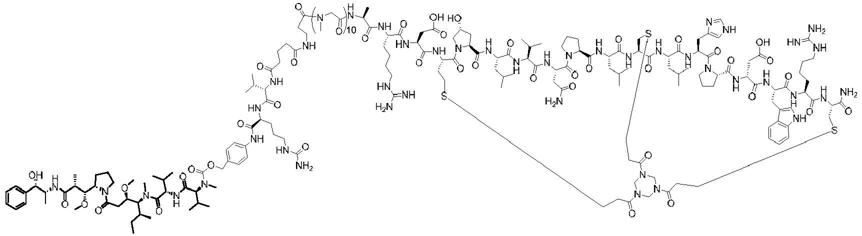

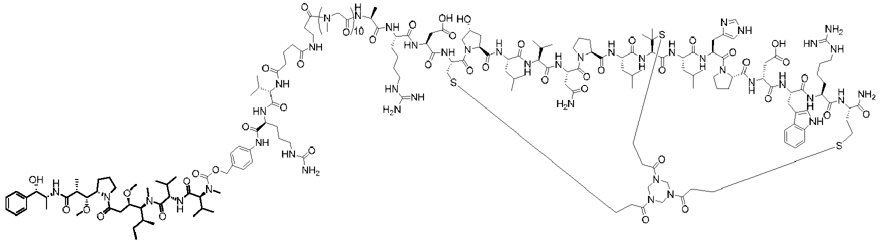

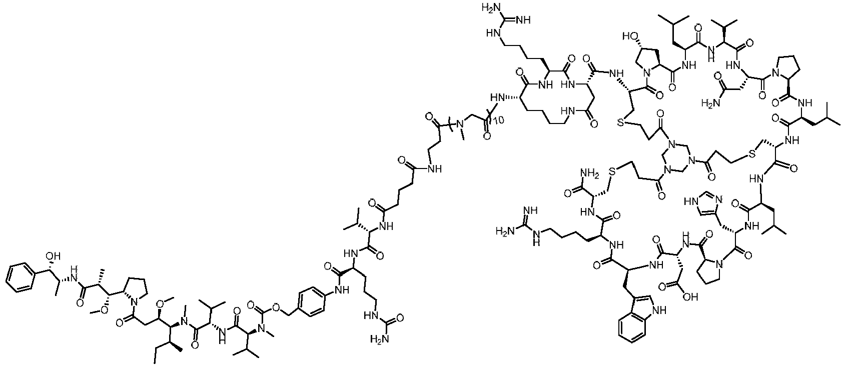

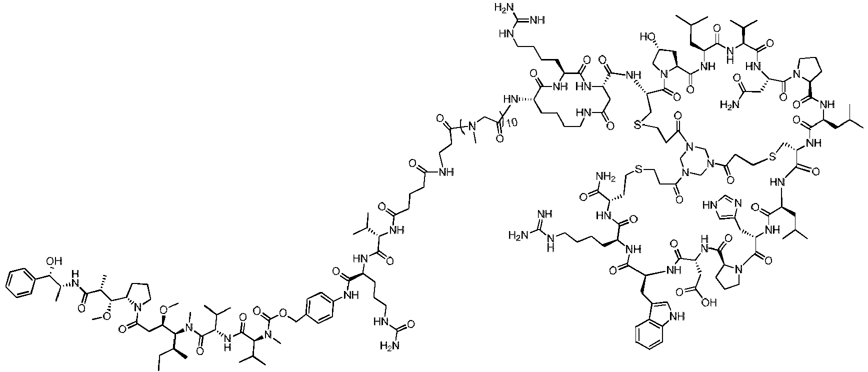

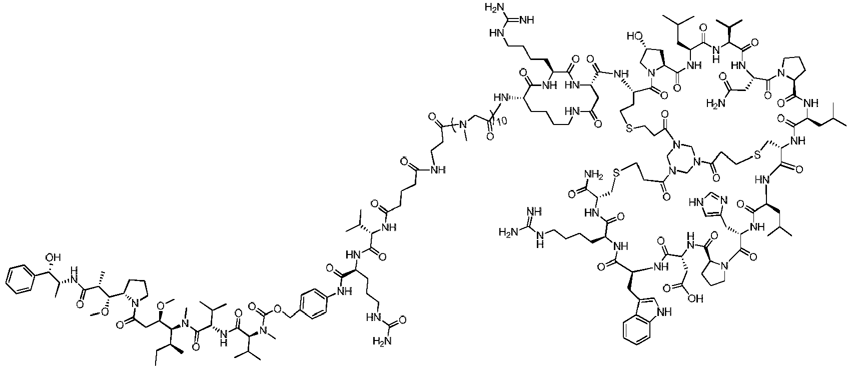

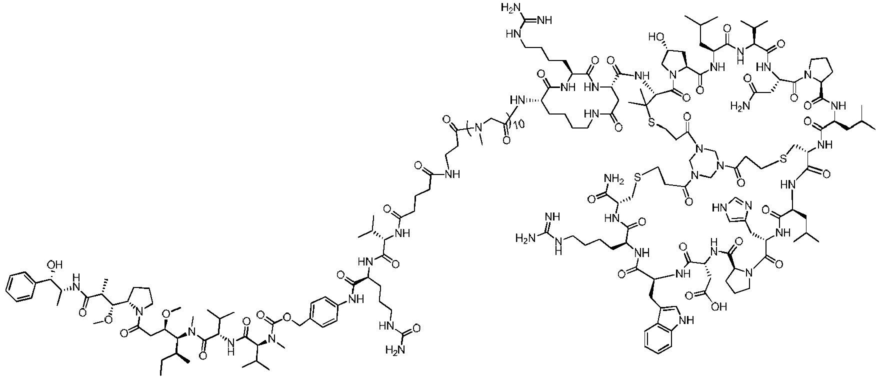

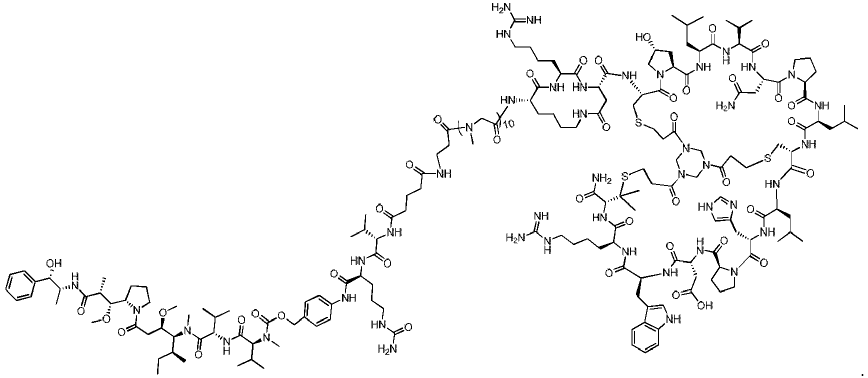

3. Use of the compounds or the pharmaceutically acceptable salts thereof according to claims 1 or 2 in preparation of a drug for treating EphA2 overexpressed solid tumors.

4. A method for treating EphA2 overexpressed solid tumors in a subject in need, comprising providing the subject with a therapeutically effective amount of the compounds or the pharmaceutically acceptable salts thereof according to claims 1 or 2.
